# FASCICULE DE BREVET EUROPEEN

(11) **EP 2 011 565 B1**
(45) Date de publication et mention de la délivrance du brevet: **03.11.2010**
(21) Numéro de dépôt: 08159742.9
(22) Date de dépôt: 04.07.2008
(51) Int. Cl.: B01J 13/08, B01J 13/14, A61K 8/11, A61K 9/50

(54) **Capsules de type noyau/écorce et procédé de préparation**
Kapseln vom Typ Kern/Wand und entsprechendes Herstellungsverfahren
Core/skin type capsules and preparation method

(30) Priorité: 05.07.2007 FR 0756298
(43) Date de publication de la demande: 07.01.2009
(73) Titulaire: L'Oréal, 75008 Paris (FR)
(72) Inventeur: Simmonet, Jean-Thierry, 94230 Cachan (FR); Biatry, Bruno, 94300 Vincennes (FR); Prigent, Fanny, 75019 Paris (FR)
(74) Mandataire: Le Coupanec, Pascale A.M.P.

(56) Documents cités:
- US-A- 3 748 277
- US-A- 3 803 045
- US-A- 5 654 008

## Description

La présente invention a pour objet un nouveau procédé de préparation d'une suspension de capsules de type noyau/écorce, enrobées ou non d'une phase lamellaire, comprenant un noyau lipidique formant ou contenant un actif lipophile et une écorce (ou enveloppe) continue, insoluble dans l'eau, comprenant au moins un polymère, en particulier un oligomère, de poids moléculaire inférieur à 10 000, non miscible à l'eau et dans le noyau lipidique, réticulé ou non.

La présente invention se rapporte également aux capsules et à une suspension de capsules susceptibles d'être obtenues selon le procédé de l'invention ainsi qu'aux compositions comprenant une telle suspension, et à leurs applications dans les domaines cosmétique, dermatologique ou pharmaceutique.

L'encapsulation ou l'absorption d'agents actifs dans des capsules de dimensions submicroniques (inférieures à 1 µm) est connue et utilisée en particulier dans les domaines cosmétique et dermatologique. En effet, ces capsules appelées nanocapsules sont capables de traverser les couches superficielles du *stratum corneum* et de pénétrer dans les couches supérieures de l'épiderme vivant pour y libérer le principe actif Cette pénétration dans des couches plus profondes de l'épiderme élargit l'espace d'action des agents actifs et les met à l'abri d'une élimination rapide par simple frottement.

Différentes techniques d'encapsulation d'agents actifs, notamment lipophiles, sont connues de l'homme du métier.

EP 0 274 961 (ou US 5,049,322), EP 0 447 318 (ou US 6,203,802) ou EP 1 029 587 (ou US 6,565,886) décrivent un procédé d'encapsulation d'agents actifs huileux ou liposolubles utilisant un solvant organique miscible à l'eau. Cependant, ce procédé présente l'inconvénient d'imposer une étape d'évaporation des solvants, et le cas échéant d'une fraction de l'eau, pour augmenter le taux d'encapsulation des agents actifs.

Qui plus est, le taux d'encapsulation demeure relativement faible, n'excédant généralement pas 8 %, et plus généralement 5 %, en poids par rapport au poids de la dispersion.

FR 2864900 (ou US 2005-175651) décrit un procédé d'encapsulation permettant un taux d'encapsulation de 10 à 15 %, mais mettant en oeuvre un solvant organique non miscible à l'eau tel que le dichlorométhane.

De tels solvants organiques peuvent être à l'origine de problèmes de sécurité et/ou de toxicité et/ou d'écotoxicité.

EP 1 462 157 décrit des microcapsules variant de 100 nm à 5 mm obtenues par un procédé de coacervation complexe résultant de l'interaction de deux polymères, l'un cationique et l'autre anionique.

US 4,124,526 décrit des particules obtenues par coacervation d'un sel d'un polymère polycarboxylique par acidification à pH 5 à 8 d'une suspension. Ce procédé présente l'inconvénient de conduire à l'obtention de particules hétérogènes en taille et possédant une taille généralement supérieure au µm, ainsi qu'une porosité élevée.

WO 01/52848 (ou US 6,451,345) décrit des microcapsules dont la taille varie de 100 à 500 µm, obtenues par coacervation d'un polymère de type éthylcellulose dans le cyclohéxane. Ce procédé présente l'inconvénient de nécessiter l'addition d'un polymère tel que le polyéthylène, pour provoquer la coacervation et ensuite d'évaporer le solvant.

WO 99/43426 (ou US 2003-180235) décrit des particules lipidiques solides entre 25 et 85 °C, de taille variant de 10 nm à 5 mm constituées d'au moins un lipide de type cire et dépourvues d'enrobage polymérique solide. Ces particules présentent l'inconvénient de présenter une stabilité limitée en association avec des agents tensioactifs.

La présente invention a pour objet de résoudre les inconvénients mentionnés ci-dessus.

La présente invention a également pour objet de proposer une suspension de capsules présentant un taux d'encapsulation d'agents actifs amélioré.

La présente invention a également pour objet de proposer un procédé de préparation d'une suspension de capsules dépourvu d'étape d'évaporation d'eau ou de solvant, en particulier de solvant organique.

La présente invention a également pour objet de proposer un procédé de préparation d'une suspension de capsules permettant de s'affranchir de l'utilisation de solvant organique.

La présente invention a également pour objet de fournir un procédé de préparation d'une suspension de capsules permettant l'obtention de capsules de faible taille, notamment inférieure à la dizaine de microns, voire inférieure au micromètre, et présentant une distribution homogène en taille.

La présente invention a également pour objet de fournir un procédé de préparation d'une suspension de capsules permettant l'obtention de capsules possédant une étanchéité améliorée.

La présente invention a encore pour objet de proposer des capsules dépourvues ou présentant une densité très faible de pores à leur surface. Ces pores sont fréquemment observés dans des capsules préparées selon des procédés comprenant une étape d'évaporation de solvant. De telles capsules présentent avantageusement une étanchéité améliorée.

Ainsi, la présente invention a également pour objet de fournir des capsules noyau/écorce présentant une étanchéité améliorée, en particulier au regard des agents actifs liposolubles encapsulés.

Ainsi, selon un de ses aspects, la présente invention a pour objet un procédé de préparation d'une suspension de capsules de type noyau/écorce comprenant au moins les étapes de :
a) disposer d'un mélange hétérogène à température ambiante (T_{amb}) comprenant :
   - une phase huileuse,
   - des polymères, identiques ou différents, non miscibles à l'eau et à la phase huileuse, dont au moins un polymère est un oligomère possédant un poids moléculaire en poids inférieur à 10 000, et un indice d'hydroxyle (I_{OH}) supérieur ou égal à 10 mg KOH/g,
b) homogénéiser la phase huileuse et les polymères par chauffage, à une température d'homogénéisation T_{H}, du mélange obtenue à l'étape a), T_{H} étant inférieure à 100 °C, ledit mélange possédant, à la température T_{H}, une viscosité inférieure ou égale à 17 mPa.s⁻¹,
c) disperser, à la température T_{H}, le mélange homogène obtenu à l'étape b) dans une phase aqueuse, pour obtenir une pré-émulsion directe dont la phase huileuse comprend les polymères,
d) soumettre la pré-émulsion obtenue à l'étape c) à un ensemble de forces de cisaillement appropriées à la réduction du diamètre des particules du mélange dispersé à une taille moyenne inférieure ou égale à environ 50 µm, et
e) refroidir l'émulsion obtenue à l'étape d) à une température T_{c} propice à la coacervation desdits polymères et à l'enrobage de gouttes de ladite phase huileuse par les coacervats,
f) refroidir la suspension obtenue à l'étape e) à une température Tp propice à la formation des capsules attendues par la précipitation et/ou la cristallisation des coacervats, la phase huileuse étant propice à la formation d'un mélange homogène avec lesdits polymères, à une température T_{H}, et à la manifestation d'un phénomène de coacervation desdits polymères à une température T_{c}.

Les inventeurs ont observé, de manière inattendue, que la mise en oeuvre de polymères comprenant au moins un ou des oligomère(s) de poids moléculaire inférieur à 10 000, d'indice d'hydroxyle (I_{OH}) supérieur ou égal à 10 mg KOH/g, et non miscible(s) à l'eau et à la phase huileuse, avec une phase huileuse apte à solubiliser au moins 10 % de ces polymères par chauffage permettait l'obtention de mélanges aptes à subir un phénomène de coacervation conduisant, après durcissement des coacervats, à l'obtention de capsules de type noyau/écorce de faible taille, homogènes en distribution de taille, et dont le noyau est formé de la phase huileuse.

En particulier, les inventeurs on montré par exemple que des oligomères de polycaprolactone (CAPA^{®}2201 ou CAPA^{®} HC 1100 de chez Solvay) avec du beurre de karité et/ou du N-lauroyle sarcosinate d'isopropyle, chauffés à 80 °C permettaient l'obtention de capsules aux propriétés requises par la présente invention.

Les conditions d'obtention des coacervats permettent avantageusement d'éviter la mise en oeuvre de solvant(s) organique(s) dans un procédé de l'invention.

Un procédé de l'invention peut permettre avantageusement d'encapsuler des quantités importantes d'agent(s) actif(s).

Par ailleurs, la préparation des coacervats de l'invention peut permettre également d'adsorber et/ou absorber des quantités importantes d'agent(s) actif(s).

Un procédé selon l'invention peut optionnellement comprendre une étape additionnelle g) de réticulation des oligomères.

Les capsules de l'invention peuvent être, ou non, enrobées d'une phase lamellaire.

Les capsules de l'invention présentent une meilleure stabilité, un taux d'encapsulation améliorée, une distribution homogène en taille, et un diamètre moyen en taille inférieur ou égal à 50 µm, en particulier inférieur ou égal à 10 µm, et notamment inférieur ou égal à 1 µm.

Au sens de l'invention, on entend par T_{amb} une température variant de 20 à 25 °C.

La présente invention a également pour objet des capsules susceptibles d'être obtenues au moyen d'un procédé de l'invention.

Ainsi, la présente invention a également pour objet des capsules de type noyau/écorce constituées :
- d'un noyau comprenant une phase huileuse, et
- d'une écorce continue insoluble dans l'eau et dans la phase huileuse, obtenue par coacervation de polymères, identiques ou différents, non miscibles à l'eau et dans la phase huileuse, et dont au moins un polymère est un oligomère possédant un poids moléculaire en poids inférieur à 10 000.

La phase huileuse peut comprendre au moins un agent actif liposoluble et/ou au moins un agent actif lipodispersible, ou peut former elle-même un agent actif.

Les capsules de l'invention sont obtenues généralement dans une suspension aqueuse, comme le montre le procédé de préparation décrit ci-dessus. Aussi, l'invention a également pour objet une suspension aqueuse de capsules de type noyau/écorce telles que décrites ci-dessus.

La présente invention a ainsi également pour objet une suspension de capsules susceptible d'être obtenue au moyen d'un procédé de l'invention.

Les capsules selon l'invention et les suspensions aqueuses les comprenant peuvent par exemple être utilisées pour la préparation de compositions cosmétiques, dermatologiques ou pharmaceutiques, par exemple à application topique.

La présente invention a également pour objet un procédé de préparation d'une composition cosmétique, dermatologique ou pharmaceutique comprenant au moins l'étape de mélanger une suspension de capsules obtenue selon le procédé selon l'invention avec un milieu physiologiquement acceptable.

L'invention a ainsi également pour objet une composition cosmétique, dermatologique ou pharmaceutique comprenant dans un milieu physiologiquement acceptable, une ou plusieurs capsules telles que décrites ci-dessus ou une suspension de capsules telle que décrite ci-dessus.

Par milieu physiologiquement acceptable, on entend un milieu compatible avec une mise en oeuvre chez un être vivant, par exemple un être humain ou un animal. En particulier un tel milieu peut être approprié à une application sur les matières kératiniques, par exemple la peau, les muqueuses, les ongles, le cuir chevelu et les cheveux.

### Capsules de type noyau/écorce

Au sens de l'invention, on entend par « capsule de type noyau/écorce », des particules ayant une structure constituée d'un coeur (ou noyau) lipidique, le cas échéant formant ou contenant un agent actif, lequel coeur est encapsulé dans une enveloppe (ou écorce) protectrice continue insoluble dans l'eau, et dans le coeur lipidique.

En d'autres termes, il s'agit selon l'invention de capsules à noyau lipidique liquide ou semi-liquide entouré d'une écorce de nature polymérique, et plus particulièrement oligomérique.

Ces particules sont par conséquent distinctes des particules de type sphère constituées d'une matrice polymérique poreuse dans laquelle l'agent actif est absorbé et/ou adsorbé.

Les capsules de l'invention possèdent avantageusement une distribution granulométrique homogène.

La distribution granulométrique qualifie la répartition de la taille des capsules autour d'une taille moyenne. La distribution granulométrique peut être caractérisée par un diamètre moyen en taille et un indice de polydispersité ou un coefficient d'uniformité. Plus la valeur de l'indice, ou du coefficient, est faible, plus les tailles des capsules se répartissent de manière homogène autour d'une taille moyenne.

Les capsules de l'invention peuvent posséder un diamètre moyen en taille inférieur ou égal à 50 µm, en particulier inférieur ou égal à 10 µm, en particulier variant de 50 nm à 10 µm, en particulier variant de 100 à 300 nm, et plus particulièrement variant de 150 à 200 nm.

Pour les capsules de taille moyenne inférieure à 1 µm (submicronique), la distribution granulométrique peut être caractérisée par un indice de polydispersité, noté IP (valeur sans dimension et caractérisant l'étendue de la distribution granulométrique) et la taille peut être exprimée en taille moyenne en intensité fournie par un granulomètre BROOKHAVEN TYPE BI90PLUS^{®} dont le principe de mesure repose sur la diffusion quasi-élastique de la lumière (QELS).

Cet indice peut varier de 0,01 à 0,35, et notamment varier de 0,05 à 0,35.

Pour les capsules de taille moyenne supérieure au micromètre, la distribution granulométrique peut être caractérisée par une taille moyenne et un coefficient d'uniformité mesurés à l'aide d'un granulomètre à diffraction laser, comme par exemple le MASTER SIZER 2000^{®} de MALVERN.

Les capsules conformes à l'invention dont la taille est supérieure au micromètre, peuvent posséder un coefficient d'uniformité avantageusement inférieur ou égal à 0,45, et de préférence supérieur ou égal à 0,1.

Selon un mode de réalisation, un procédé de l'invention peut conduire à l'obtention d'une suspension de capsules de l'invention pouvant présenter un taux en capsules variant de 3 à 90 %, en particulier variant de 10 à 60 %, et plus particulièrement variant de 20 à 50 %, en poids par rapport au poids total de la suspension.

Selon un mode de réalisation, une suspension de capsules de l'invention peut comprendre jusqu'à 80 % en poids d'agent actif encapsulé par rapport au poids total d'agent actif mis en oeuvre à l'étape a) du procédé décrit ci-dessus, en particulier jusqu'à 85 %, en particulier jusqu'à 90 %, et plus particulièrement jusqu'à 95, voire jusqu'à 99 % d'agent actif encapsulé par rapport au poids total d'agent actif mis en oeuvre dans le mélange initial de l'étape a) du procédé ci-dessus.

Les capsules obtenues par un procédé de l'invention possèdent avantageusement une étanchéité améliorée.

L'étanchéité des capsules de l'invention peut être mesurée par toute technique connue de l'homme de l'art dans le domaine, telle que la mesure de la fuite hors des capsules d'un marqueur colorée ou fluorescent préalablement encapsulé.

### Procédé

Ainsi comme indiqué ci-dessus, la présente invention a pour objet un procédé de préparation d'une suspension de capsules de type noyau/écorce comprenant au moins les étapes de :
a) disposer d'un mélange hétérogène à température ambiante (T_{amb}) comprenant :
   - une phase huileuse,
   - des polymères, identiques ou différents, non miscibles à l'eau et à la phase huileuse, dont au moins un polymère est un oligomère possédant un poids moléculaire en poids inférieur à 10 000, et un indice d'hydroxyle (I_{OH}) supérieur ou égal à 10 mg KOH/g,
b) homogénéiser la phase huileuse et les polymères par chauffage, à une température d'homogénéisation T_{H}, du mélange obtenue à l'étape a), T_{H} étant inférieur à 100 °C, ledit mélange ayant, à la température T_{H}, une viscosité inférieure à 17 mPa.s⁻¹,
c) disperser, à la température T_{H}, le mélange homogène obtenu à l'étape b) dans une phase aqueuse, pour obtenir une pré-émulsion directe dont la phase huileuse comprend les polymères,
d) soumettre la pré-émulsion obtenue à l'étape d) à un ensemble de forces de cisaillement appropriées à la réduction du diamètre des particules du mélange dispersé à une taille moyenne inférieure ou égale à environ 50 µm, et
e) refroidir l'émulsion obtenue à l'étape c) à une température T_{c} propice à la coacervation desdits polymères et à l'enrobage de gouttes de ladite phase huileuse par les coacervats,
f) refroidir la suspension obtenue à l'étape e) à une température Tp propice à la formation des capsules attendues par la précipitation et/ou la cristallisation des coacervats,
la phase huileuse étant propice à la formation d'un mélange homogène, avec lesdits polymères, à une température T_{H} et à la manifestation d'un phénomène de coacervation desdits polymères à une température T_{c}.

Selon un mode de réalisation particulier, les polymères mis en oeuvre à l'étape a) sont des oligomères possédant un poids moléculaire en poids inférieur à 10 000, et un indice d'hydroxyle (I_{OH}) supérieur ou égal à 10 mg KOH/g.

Le mélange obtenu à l'étape a) est effectué à température ambiante, variant de 20 à 25°C, permettant d'observer le caractère hétérogène du mélange.

Pour obtenir un tel mélange, les polymères et la phase huileuse sont sélectionnés de telle sorte que :
- un mélange phase huileuse et polymères, possède une température d'homogénéisation T_{H} inférieure à 100 °C, et supérieure à la température ambiante T_{amb},
- ce mélange possède une température de coacervation T_{c} inférieure à T_{H}, et supérieure à T_{amb},
- ce mélange possède, à la température T_{H}, une viscosité inférieure ou égale à 17 mPa.s-¹, et
- les polymères, à température ambiante, sont non miscibles à l'eau et à la phase huileuse.

Selon un mode de réalisation, la phase huileuse peut comprendre au moins un agent actif liposoluble et/ou au moins un agent actif lipodispersible.

Selon un mode de réalisation la phase huileuse peut être préalablement mélangée avec le(s) agent(s) actif(s) à encapsuler, avant addition des polymères.

Selon une variante de réalisation, la phase huileuse, le(s) agent(s) actif(s) et les polymères peuvent être mélangés simultanément.

Les agents actifs liposolubles susceptibles d'être mis en oeuvre dans l'invention sont choisis et mis en oeuvre en des teneurs de telle sorte que les propriétés du mélange tripartite ainsi obtenu (phase huileuse/polymères/agents actifs liposolubles) soient conformes aux propriétés requises décrites ci-dessus pour le mélange bipartite obtenu à l'étape a) (phase huileuse/polymères).

Au sens de l'invention, on entend par mélange hétérogène, un mélange comprenant au moins deux phases différentes, visibles à l'oeil nu.

Le mélange hétérogène ainsi obtenu à l'étape a) est ensuite chauffé à une température d'homogénéisation T_{H} du mélange. Une telle température peut être aisément déterminée par des expériences de routine connues de l'homme de l'art.

Les moyens de chauffage convenant à la mise en oeuvre de l'invention sont habituellement utilisés par l'homme du métier dans le domaine.

Des exemples de polymères, en particulier d'oligomères, de phase huileuse, et le cas échéant d'agent(s) actif(s) convenant à l'invention sont notamment présentés par la suite.

La température d'homogénéisation d'un mélange de l'invention est supérieure ou égale à la température à laquelle les polymères, en particulier les oligomères deviennent miscibles avec la phase huileuse, et inférieure à 100 °C.

Ainsi à l'issu de l'étape b) on obtient un mélange homogène monophasique.

Au regard de l'invention, le caractère homogène d'un tel mélange s'apprécie vis-à-vis de la phase huileuse et des polymères, en particulier des oligomères et, le cas échéant, des agents actifs liposolubles.

Selon une variante de réalisation, l'homogénéité d'un mélange de l'invention comprenant au moins un agent actif lipodispersible s'apprécie d'une part par l'homogénéité du mélange phase huileuse/oligomères, et d'autre part, l'homogénéité de la dispersion des agents actifs lipodispersibles.

La miscibilité des polymères, en particuliers des oligomères de l'invention, dans une phase huileuse appropriée au procédé de l'invention, comprenant éventuellement un ou des agent(s) actif(s) liposoluble(s), ainsi que, le cas échéant, la dispersion homogène des agents actifs lipodispersibles peuvent, par exemple, être appréciées à l'oeil nu.

Le mélange obtenu à l'étape b) possède à la température T_{H} une viscosité inférieure ou égale à 17 mPa.s⁻¹.

La viscosité d'un mélange de l'invention peut être mesurée à l'aide d'un rhéomètre RS150 Rhéostress de chez KAAKE équipé d'une géométrie cône-plan (60 mm de diamètre, 2° d'angle, en titane) sous écoulement (balayage en gradient de cisaillement de 1 à 1000 ^{s-1}).

La valeur de viscosité relevée est celle sur le plateau newtonien à faible gradient de cisaillement.

Par exemple, un mélange obtenu à l'étape b) peut posséder, à la température T_{H}, une viscosité variant de 1 à 17 mPa.s-¹.

A l'étape c) le mélange monophasique est ensuite dispersé dans une phase aqueuse chauffée à une température telle que la température du mélange ainsi obtenu soit supérieure ou égale à la température T_{H}, de manière à obtenir une pré-émulsion de gouttes du mélange dans une phase aqueuse continue.

Selon un mode de réalisation, la phase aqueuse peut être chauffée à une température équivalente à la température T_{H} déterminée ci-dessus.

Selon un mode de réalisation, comme indiqué ci-après, un ou des agents tensioactifs tels que définis ci-après peuvent être introduits dans la phase huileuse et/ou dans la phase aqueuse, avant la préparation de la pré-émulsion.

Le cas échéant le(s) agent(s) tensioactif(s) additionnel(s) peu(ven)t être mis en oeuvre pour l'obtention d'une phase lamellaire destinée à entourer les capsules de l'invention.

Les dispositifs permettant de préparer une pré-émulsion selon l'invention sont ceux habituellement mis en oeuvre par l'homme du métier dans le domaine.

La taille finale attendue des capsules est généralement déterminante pour le choix du dispositif et des forces à mettre en oeuvre lors de la préparation de la pré-émulsion. Ainsi, le dispositif et les conditions de préparation de la pré-émulsion de l'étape c) sont adaptés par l'homme du métier au regard de la taille des capsules de l'invention à obtenir.

La pré-émulsion obtenue à l'étape c) peut être soumise à l'étape d) à des forces de cisaillement suffisantes pour obtenir des gouttes du mélange dispersé au plus égales à 50 µm en diamètre, en particulier au plus égales à 10 µm en diamètre.

L'étape d) peut être mise en oeuvre par tout dispositif connu de l'homme de l'art apte à la réduction de la taille des globules de la pré-émulsion à la taille attendue pour les capsules de l'invention, notamment inférieure ou égale à 50 µm.

A titre d'exemple de dispositif convenant à l'invention on peut mentionner les homogénéisateurs à haute pression ou à ultrasons.

L'émulsion obtenue à l'étape d) est alors au moins refroidie à une température de coacervation T_{c}, sous faible agitation (étape e)).

La température de coacervation T_{c} est la température à laquelle les polymères, et en particulier les oligomères, s'organisent en coacervats, c'est-à-dire sous la forme d'écorces molles, autour des gouttelettes de la phase huileuse. T_{c} est supérieure à T_{amb}.

Avantageusement, T_{c} peut être supérieure ou égale à 45 °C.

La suspension de particules huileuses enrobées d'un coacervat d'oligomères est ensuite soumise à une étape de refroidissement f) jusqu'à une température Tp propice à la formation des capsules attendues par la précipitation et/ou la cristallisation des coacervats.

La température Tp est inférieure au point de fusion P_{f} le plus bas des oligomères mis en oeuvres dans un procédé de l'invention.

Selon un mode de réalisation, les polymères, et en particulier les oligomères, sont avantageusement choisis de sorte que P_{f} soit supérieur à T_{amb}. Avantageusement lorsque P_{f} est supérieur à T_{amb} on peut choisir Tₚ égale à T_{amb}.

Selon un autre mode de réalisation, les polymères, et en particuliers les oligomères, peuvent être choisis de sorte que P_{f} soit inférieur à T_{amb}. Avantageusement lorsque P_{f} est inférieur à T_{amb} on peut effectuer une étape g) additionnelle de réticulation comme décrit ci-après.

Les étapes e) et f) de refroidissement d'une émulsion de l'invention peuvent être réalisées de façon continue ou par étape.

On entend par « refroidissement continu », le maintien de l'émulsion à refroidir pendant une période de temps identique à chaque fraction déterminée et identique de température inférieure.

On entend par « refroidissement par étape », le maintien de l'émulsion à refroidir à une fraction de température inférieure pendant une période de temps donnée.

Selon un mode de réalisation un mélange selon l'invention contenant une phase huileuse, comprenant le cas échéants le(s) agent(s) actif(s), et des polymères, en particulier des oligomères, est formulé de telle sorte que la température T_{c} est supérieure ou égale à 45 °C.

Selon une variante de réalisation, on peut introduire dans le processus de refroidissement, avant ou après T_{c} et Tp, autant d'étapes que souhaitées.

Selon une variante de réalisation, la vitesse de refroidissement peut être constante, accélérée ou se ralentissant de T_{H} à T_{c} et/ou de T_{c} à Tₚ.

Selon un autre mode de réalisation, le refroidissement de l'émulsion de l'invention peut être effectué sans étape de T_{H} à Tp.

Le refroidissement peut être opéré en confinant l'émulsion ainsi obtenue dans une enceinte dont la température peut être fixée de manière à régler la vitesse de refroidissement, et les différentes étapes de refroidissement.

Selon une variante de réalisation le refroidissement peut être opéré en plaçant l'émulsion obtenue à l'étape d) dans une atmosphère maintenue à température ambiante.

A l'issu de l'étape de refroidissement, on obtient alors une suspension de capsules dont on peut contrôler la taille à l'aide d'un granulomètre laser à diffusion de la lumière ou d'un granulomètre à diffraction laser, comme indiqué ci-dessus.

Selon un mode de réalisation, un procédé selon l'invention peut comprendre en outre une étape g) de réticulation des oligomères.

L'étape, optionnelle, de réticulation peut permettre de renforcer la paroi des capsules de l'invention. Une telle étape permet avantageusement d'améliorer l'étanchéité des capsules de l'invention.

L'étape de réticulation peut être effectuée au moyen d'agent(s) de réticulation tels que définis ci-après.

Selon un mode de réalisation, le(s) agent(s) de réticulation est/sont ajouté(s) à l'émulsion de l'invention à l'issu de l'étape de refroidissement.

Selon un mode de réalisation, l'étape de réticulation g) peut être en particulier mise en oeuvre lorsque le ou les polymères, en particulier le ou les oligomère(s), utilisé(s) dans le procédé de l'invention possède(nt) un point de fusion P_{f} inférieur ou égale 45 °C, en particulier inférieur ou égale à T_{amb}, et notamment est/sont liquide(s) à température ambiante.

Selon un mode de réalisation, les polymères, en particulier les oligomères, et la phase huileuse, comprenant optionnellement un ou de(s) agent(s) actif(s), peuvent être présents selon un rapport pondéral variant de 5/1 à 1/20, en particulier variant de 2/1 à 1/20, et plus particulièrement variant de 2/1 à 1/10.

Selon un mode de réalisation, une phase huileuse et le(s) agent(s) actif(s) peuvent être présents dans un mélange hétérogène selon l'invention selon un rapport pondéral variant de 100/0 à 1/99.

Selon un mode de réalisation, la phase huileuse peut former l'agent actif.

### Polymères

Au sens de l'invention, on entend par polymères, des composés comprenant au moins 2, en particulier au moins 3, et plus particulièrement au moins 4 motifs identiques répétés.

Au sens de l'invention, on entend par « oligomère », des polymères comportant au plus 100 unités monomériques.

Les oligomères convenant à l'invention peuvent posséder un poids moléculaire en poids inférieur à 10 000, et supérieur ou égal à 400.

En particulier, ils peuvent posséder un indice d'hydroxyle (I_{OH}) au moins égal à 10 mg KOH/g, en particulier variant de 10 à 700 mg KOH/g, en particulier variant de 10 à 600 et plus particulièrement variant de 40 à 500 mg KOH/g.

Dans une mise en oeuvre d'un procédé de l'invention, les oligomères peuvent être identiques ou différents.

Les polymères convenant à l'invention, et en particulier les oligomères, sont non miscibles à l'eau et à la phase huileuse destinée à former le noyau des capsules de l'invention. Ils possèdent un point de fusion P_{f} inférieur à 100 °C, en particulier variant de -20 °C à 95 °C, et plus particulièrement variant de -10°C à 80 °C.

Selon un mode de réalisation, on peut mettre en oeuvre à l'étape a), en mélange avec des oligomères possédant un poids moléculaire en poids inférieur à 10 000, des polymères possédant un poids moléculaire en poids supérieur ou égal à 10 000, notamment supérieur ou égal à 15 000.

En particulier les polymères de poids moléculaire supérieur ou égal à 10 000 possèdent un poids moléculaire ne dépassant pas 80 000

Selon une variante de réalisation, les polymères mis en oeuvre dans un procédé de l'invention possèdent tous un poids moléculaire en poids inférieur à 10 000.

A titre illustratif et non limitatif d'oligomères convenant à l'invention, on peut par exemple, citer les oligomères choisis parmi les polycaprolactones, les poly-L-lactides, les poly-DL-lactides, les polyglycolides et leurs copolymères, les polymères de l'acide 3-hydroxybutyrique et ses copolymères avec l'acide hydroxyvalérique, les polycarbonates diols, les poly(alkylène adipate), les polyesters polyols, les polyesters dendritiques à fonction hydroxyle terminale, et leurs mélanges.

Les oligomères convenant à l'invention peuvent, par exemple, être choisis parmi :
- les polycaprolactones, par exemple ceux commercialisés par SOLVAY qui peuvent être obtenus à partir d'un diol, d'un triol, d'un tétrol, ou d'un glycol carboxylé.

Les diols utilisés pour la préparation de ces oligomères de polycaprolactone sont : le 1,4-butanediol, le 1,6-hexanediol, le mono ou diéthylène glycol, le 2,2-dimethyl-1,3-propanediol (neopentyl glycol), le diméthylol acide propionique. Les triols peuvent être par exemple un mélange de diéthylène glycol et de glycérol, le trimethylol propane
- les poly-L-lactides, les poly-DL-lactides, les polyglycolides et leurs copolymères,
- les polymères de l'acide 3-hydroxybutyrique et leurs copolymères avec l'acide hydroxyvalérique,
- les polycarbonate diols tels que les hexanediol polycarbonates, par exemple le UH-Carb 50 et 300 commercialisé par UBE
- les poly(alkylène adipate) comprenant les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols ; les alcane-diols utilisés pour la préparation desdits poly(alkylène adipate) peuvent être des alcane-diols en C₂-C₆ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol. Les éther-diols sont des di-, tri- ou tétra-(alkylène en C₂-C₄)-glycols tels que le diéthylèneglycol, le triéthylèneglycol, le tétraéthylèneglycol, le dipropylèneglycol, le tripropylèneglycol, le tétrapropylèneglycol, le dibutylèneglycol, le tributylèneglycol ou le tétrabutylèneglycol ; à titre d'exemple, on peut citer les Fomrez^{®} commercialisés par la société Witco et les poly(éthylène adipate) de la société Scientific Polymer Products ; on peut également citer à titre d'exemple, l'Eastar Bio^{®} de Eastman Chemical (poly(tetraméthylène adipate co-téréphtalate) et l'Ecoflex F BX 7011^{®} de BASF (terpolymère butanediol 1,4 acide terephtalique et acide adipique).

A titre d'exemple de poly(alkylene adipate) convenant à l'invention, on peut en particulier citer les FOMREZ^{®} de la société Witco.
- les polyesters polyols, tels que les copolymères obtenus par polycondensation d'au moins un acide dicarboxylique aliphatique, avec au moins deux alcane-diols, ou avec au moins un alcane-diol et au moins un hydroxyalkyl alcane-diol), et, éventuellement, une faible proportion de triols.

L'acide dicarboxylique aliphatique utilisé pour la préparation desdits polyesters polyols peut être choisi par exemple parmi l'acide malonique, l'acide succinique, l'acide glutarique, l'acide adipique (ou acide hexane-1,6-dioïque), l'acide pinélique, l'acide sébacique, l'acide azélaique et leurs mélanges. Selon un mode de réalisation de l'invention, l'acide dicarboxylique peut être l'acide adipique.

Les alcane-diols utilisés pour la préparation des polyesters polyols peuvent être choisis parmi des alcane-diols à chaîne linéaire ou ramifiée comportant de 2 à 20 atomes de carbone, et de préférence de 2 à 10 atomes de carbone. Ils peuvent être choisis notamment parmi l'éthylèneglycol, le propylèneglycol, le 1,3-propanediol, le 1,4-butanediol, le 1,5-pentanediol, le 1,6-hexanediol, le néopentylglycol et leurs mélanges. Selon un mode de réalisation de l'invention, l'alcane-diol peut être choisi parmi le 1,4-butanediol, le 1,6-hexane diol et leurs mélanges. Par exemple, l'alcane-diol peut être le 1,4-butanediol. 1.

On entend dans la présente demande par « hydroxyalkyl alcane-diols comportant éventuellement une chaîne alkyle », des alcane-diols comportant au moins un groupe hydroxyalkyle, et pouvant en outre comporter une chaîne alkyle, où le groupe hydroxyalkyle et la chaîne alkyle sont, indépendamment l'un de l'autre, des chaînes saturées, linéaires ou ramifiées, comportant de 1 à 10 atomes de carbone. Les hydroxyalkyl alcane-diols utilisables pour former les polyesters polyols de la présente invention peuvent être choisis par exemple parmi les 2-alkyl-2-(hydroxyalkyl)-1,3-propanediol, où le groupe hydroxyalkyle et la chaîne alkyle comportent, indépendamment l'un de l'autre, de 1 à 10 atomes de carbone, tels que par exemple le 2-éthyl-2-(hydroxyméthyl)-1,3-propanediol et le 2-méthyl-2-(hydroxyméthyl)-1,3-propanediol ; les 2-(hydroxyalkyl)-1,3-propanediol où le groupe hydroxyalkyle comporte de 1 à 10 atomes de carbone ; et leurs mélanges.

Selon un mode de réalisation de invention, on peut utiliser comme hydroxyalkyl alcane-diol, le 2-éthyl-2-(hydroxyméthyl)-1,3-propanediol.

Les polyesters polyols convenant à l'invention peuvent également contenir un nombre limité de motifs de ramification dérivés de triols. Les triols utilisés peuvent être choisis parmi le glycérol, le triméthyloléthane et le triméthylolpropane. La fraction des motifs de ramification dérivés des triols ci-dessus n'excède généralement pas 5 % en moles par rapport à l'ensemble des motifs dérivés de diols et de triols.

Selon un mode de réalisation de la présente invention, les polyester polyols peuvent être choisi parmi les polyesters polyols obtenus à partir d'acide adipique, de 1,4-butanediol, et de 1,6-hexane diol et les polyesters polyols obtenus à partir d'acide adipique, de 1,4-butanediol et de 2-éthyl-2-(hydroxyméthyl)-1,3-propanediol. Comme polyesters polyols convenant à l'invention, on peut citer par exemple ceux commercialisés par la société INOLEX sous les dénominations LEXOREZ. Sont particulièrement préférés, ceux obtenus à partir d'acide adipique, de 1,4-butanediol et de 2-éthyl-2-(hydroxyméthyl)-1,3-propanediol.

Les polyesters polyols convenant à l'invention peuvent posséder un point de fusion allant de -20 °C à 95 °C et notamment de -10 °C à 80 °C.

Les polyesters polyols convenant à l'invention peuvent être préparés selon des procédés habituellement utilisés pour la préparation des polyesters.

Les polyesters polyols convenant à l'invention peuvent être notamment ceux commercialisés sous la dénomination LEXOREZ® par la société INOLEX ainsi que les polyesters polyols commercialisés sous la dénomination Eternacoll tel que l'Eternacoll 3020 de chez UBE.
- les copolymères polylactate ricinoléate, tels que ceux commercialisés sous la marque ETHOX PLPR par la société ETHOX CHEMICAL, et les copolymères polylactate hydroxystéarate, notamment ceux commercialisés sous la marque ETHOX PLPHS par la société EHTOX CHEMICAL,
- les polyesters dendritiques à fonction hydroxyle terminale.

Les polymères dendritiques ou dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures moléculaires construites autour d'un motif central généralement polyvalent. Autour de ce motif central sont enchaînés, en couches concentriques et selon une structure parfaitement déterminée, des motifs ramifiés d'allongement de chaîne donnant ainsi naissance à des macromolécules symétriques, monodispersées ayant une structure chimique et stéréochimique bien définie.

Les polymères dendritiques convenant à l'invention sont des polymères hyperramifiés ayant la structure chimique d'un polyester et qui sont terminés par des groupements hydroxyle éventuellement modifiés par au moins un agent de terminaison de chaîne. La structure et la préparation de tels polymères est décrite dans les demandes de brevet WO-A-93/17060 (ou son équivalent US 5,418,301) et WO 96/12754 (ou son équivalent US 5,663,247).

Les polymères dendritiques utilisés dans la présente invention peuvent être définis comme étant des macromolécules hautement ramifiées de type polyester, constituées :
- d'un motif central dérivé d'un composé initiateur portant une ou plusieurs fonctions hydroxyle (a),
- de motifs d'allongement de chaîne dérivés d'une molécule d'allongement de chaîne portant une fonction carboxyle (b) et au moins deux fonctions hydroxyle (c),
chacune des fonctions hydroxyle (a) de la molécule centrale étant le point de départ d'une réaction de polycondensation (par estérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

On appelle un dendrimère de "génération X" un polymère hyperramifié préparé par X cycles de condensation, chaque cycle consistant à faire réagir l'ensemble des fonctions réactives du motif central ou du polymère avec un équivalent d'une molécule d'allongement de chaîne.

Le composé initiateur portant une ou plusieurs fonctions hydroxyle et formant le motif central autour duquel se construira la structure dendritique est un composé mono-, di- ou polyhydroxylé. Il peut être choisi parmi :
(a) un alcool mono fonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un α-alkylglycoside,
(h) un polymère polyalcoxylé obtenu par polyalcoxylation d'un des alcools (a) à (g), ayant une masse molaire au plus égale à 8000.

On peut citer à titre d'exemples de composés initiateurs propices à la préparation des polyesters dendritiques convenant à l'invention, le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

On fait réagir sur ces composés initiateurs hydroxylés formant le motif central du futur dendrimère, des molécules dites molécules d'allongement de chaîne qui sont des composés de type diol-monoacide choisis parmi :
- les acides monocarboxyliques comportant au moins deux fonctions hydroxyle, et
- les acides monocarboxyliques comportant au moins deux fonctions hydroxyle dont une ou plusieurs porte(nt) un substituant hydroxyalkyle.

Des exemples de tels composés sont l'acide diméthylolpropionique, l'acide α,α-bis(hydroxyméthyl)-butyrique, l'acide α,α,α-tris(hydroxyméthyl)-acétique, l'acide α,α-bis(hydroxyméthyl)-valérique, l'acide α,α-bis(hydroxy)-propionique et l'acide 3,5-dihydroxybenzoïque.

Selon un mode de réalisation de l'invention, le composé initiateur peut être choisi parmi le triméthylolpropane, le pentaérythritol et un pentaérythritol éthoxylé, et la molécule d'allongement de chaîne est l'acide diméthylolpropionique.

Une partie des fonctions hydroxyle terminales des polymères dendritiques de type polyester peuvent porter des substituants dérivés d'au moins un agent de terminaison de chaîne.

La fraction de ces fonctions hydroxyle terminales portant un motif de terminaison de chaîne est généralement comprise entre 1 et 90 % en moles, en particulier entre 10 et 50 % en moles rapporté au nombre total de fonctions hydroxyle terminales.

Le choix d'un agent de terminaison de chaîne approprié permet de modifier à souhait les propriétés physico-chimiques des polyesters dendritiques utilisés dans l'invention.

Ledit agent de terminaison de chaîne peut être choisi dans une grande variété de composés capables de former des liaisons covalentes avec les fonctions hydroxyle terminales.

Ces composés peuvent comprendre :
i) les acides (ou anhydrides) monocarboxyliques aliphatiques ou cycloaliphatiques saturés ou insaturés,
ii) les acides gras saturés ou insaturés,
iii) les acides monocarboxyliques aromatiques,
iv) les diisocyanates monomères ou oligomères ou des produits d'addition de ceux-ci,
v) les épihalogénhydrines,
vi) les esters glycidyliques d'un acide monocarboxylique ou d'un acide gras en C₁₋₂₄,
vii) les éthers glycidyliques d'alcools monovalents en C₁-₂₄,
viii) les produits d'addition dérivés d'un acide mono-, di- ou polycarboxylé aliphatique ou cycloaliphatique, saturé ou insaturé, ou des anhydrides correspondants,
ix) les produits d'addition dérivés d'un acide mono-, di- ou polycarboxylé aromatique ou des anhydrides correspondants,
x) les époxydes d'un acide monocarboxylique insaturé en C₃₋₂₄ ou d'un triglycéride correspondant,
xi) les alcools monofonctionnels aliphatiques ou cycloaliphatiques, saturés ou insaturés,
xii) les alcools monofonctionnels aromatiques,
xiii) les produits d'addition dérivés d'un alcool mono-, di- ou polyfonctionnel, aliphatique ou cycloaliphatique, saturé ou insaturé, et
xiv) les produits d'addition dérivés d'un alcool mono-, di- ou polyfonctionnel aromatique.

On peut citer à titre d'exemple d'agents de terminaison de chaîne l'acide laurique, les acides gras de graines de lin, les acides gras de soja, les acides gras de suif, les acides gras d'huile de ricin déshydrogénée, l'acide crotonique, l'acide caprique, l'acide caprylique, l'acide acrylique, l'acide méthacrylique, l'acide benzoïque, l'acide para-tert-butylbenzoïque, l'acide abiétique, l'acide sorbinique, le 1-chloro-2,3-époxypropane, le 1,4-dichloro-2,3-époxybutane, les acides gras de soja époxydés, le maléate de l'éther diallylique de triméthylolpropane, le 5-méthyl-1,3-dioxane-5-méthanol, le 5-éthyl-1,3-dioxane-5-méthanol, l'éther diallylique de triméthylolpropane, l'éther triallylique de pentaérythritol, le triacrylate de pentaérythritol, le triacrylate de pentaérythritol triéthoxylé, le toluène-2,4-diisocyanate, le toluène-2,6-diisocyanate, l'hexaméthylène-diisocyanate ou l'isophorone-diisocyanate.

Parmi ces agents de terminaison de chaîne, on peut citer en particulier l'acide caprique et l'acide caprylique ou un mélange de ceux-ci.

Les polymères dendritiques de type polyester à fonctions hydroxyle terminales et portant éventuellement des groupements de terminaison de chaîne sont connus et sont commercialisés par la société PERSTORP.

Des polymères convenant à la présente invention peuvent être :
- un polyester dendritique obtenu par polycondensation d'acide diméthylolpropionique sur du triméthylolpropane et exempt d'agents de terminaison de chaîne, par exemple celui commercialisé sous la dénomination "BOLTORN® H40 (TMP core)" par la société PERSTORP ;
- un polyester dendritique obtenu par polycondensation d'acide diméthylolpropionique sur du pentaérythritol polyoxyéthyléné (en moyenne 5 motifs d'oxyde d'éthylène sur chaque fonction hydroxyle), exempt d'agent de terminaison de chaîne par exemple celui commercialisé sous la dénomination "BOLTORN® H30" par la société PERSTORP ;
- un polyester dendritique de génération 3 obtenu par polycondensation d'acide diméthylolpropionique sur du pentaérythritol polyoxyéthyléné (en moyenne 5 motifs d'oxyde d'éthylène sur chaque fonction hydroxyle), dont 50 % des fonctions hydroxyle sont estérifiées par des acides en C₈₋₁₀ et notamment les acides caprique(C₁₀) et caprylique(C₈), ("BOLTORN® H30 (estérifié)" vendu par la société PERSTORP).

A titre de polyesters dendritiques à fonction hydroxyle terminale convenant notamment à l'invention, on peut citer les oligomères commercialisés sous la dénomination BOLTORN® de la société PERSTORP).

Parmi les oligomères convenant à l'invention, on peut en particulier citer les polycaprolactones tels que le Capa^{®} HC 1100 de poids moléculaire de 1000, d'indice d'hydroxyle (mg KOH/g) de 110 et de point de fusion (°C) de 45-50°C, le Capa^{®} 2200A et le Capa^{®} 2201 de poids moléculaire de 2000, d'indice d'hydroxyle (mg KOH/g) de 56 et de point de fusion (°C) de 40-50, le Capa^{®} 2302A et le Capa^{®} 2303 de poids moléculaire de 3000, d'indice d'hydroxyle (mg KOH/g) de 37 et de point de fusion (°C) de 50-60, le Capa^{®} 3050 de poids moléculaire de 540, d'indice d'hydroxyle (mg KOH/g) de 310 et de point de fusion (°C) de 0-10 ; les poly(alkylène adipate) tels que le Fomrez^{®} F930 de poids moléculaire de 2000, d'indice d'hydroxyle (mg KOH/g) de 54-58 et de point de fusion (°C) de 50 ; les polyesters polyols tels que le Lexorez^{®} 1151-35 de poids moléculaire de 3200, d'indice d'hydroxyle (mg KOH/g) de 35 et de point de fusion (°C) de 55-65, le Lexorez^{®} 1460-36 de poids moléculaire de 3200, d'indice d'hydroxyle (mg KOH/g) de 36 et de point de fusion (°C) de 40-50, l'UH-CARB^{®} 300 de poids moléculaire de 3000, d'indice d'hydroxyle (mg KOH/g) de 37 et de point de fusion (°C) de 52, l'UH-CARB^{®} 50 de poids moléculaire de 500, d'indice d'hydroxyle (mg KOH/g) de 224 et de point de fusion (°C) de 33 et l'Eternacoll^{®} 3020 de poids moléculaire de 3500, d'indice d'hydroxyle (mg KOH/g) de 29-35 et de point de fusion (°C) de 65.

A titre d'exemple de polymères possédant un poids moléculaire en poids supérieur ou égal à 10 000, et notamment supérieur ou égal à 15 000, on peut citer les polymères choisis parmi :
- les polymères de cyanoacrylate d'alkyle en C₂-C₁₂ et, en particulier, en C₂-C₆ avec le radical alkyle pouvant en particulier choisi parmi les radicaux éthyle, n-butyle, hexyle, isobutyle et isohexyle,
- les polymères formés par les poly-L-lactides, les poly-DL-lactides, les polyglycolides et les copolymères correspondants, tels que les copoly(DL-lactides et glycolides), copoly(glycolides et caprolactones) et similaires,
- les polycaprolactones, telles que les polycaprolactones ayant un point de fusion variant de 40 à 70 °C et de poids moléculaire compris entre 2000 et 100000, comme par exemple celles commercialisées par la société Solvay sous les références commerciales CAPA 6806 (PM de 80000), CAPA 6100 (PM de 10000), CAPA 6506 (PM 50000), CAPA 6250 (PM de 25000), CAPA 2803 (PM de 8000), CAPA 2403 D (PM de 4000),
- les polymères de l'acide 3-hydroxybutyrique et ses copolymères avec l'acide hydroxyvalérique,
- les copolymères de chlorure de vinyle et d'acétate de vinyle, par exemple ceux commercialisés sous la dénomination RHODOPAS AX 8515^{®} par la société RHONE POULENC,
- les copolymères d'acide et d'ester méthacrylique, notamment d'acide méthacrylique et d'ester d'acide méthacrylique, par exemple ceux commercialisés sous la dénomination EUDRAGIT L 100^{®} par la société ROHM PHARMA,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylèneglycols,
- les poly-(méthacrylate d'hydroxyalkyle en C₁ à C₄) et en particulier les poly-(méthacrylate d'hydroxyéthyle),
- les dérivés de cellulose tels que les esters de cellulose et d'au moins un acide carboxylique en C₁ à C₄, notamment des esters de cellulose mixtes de deux types d'acides carboxyliques,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly(alkylène adipate) qui englobe à la fois les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols ; les alcane-diols utilisés pour la préparation desdits poly(alkylène adipate) peuvent être des alcane-diols en C₂-C₆ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol. Les éther-diols sont des di-, tri- ou tétra-(alkylène en C₂-C₄)-glycols tels que le diéthylèneglycol, le triéthylèneglycol, le tétraéthylèneglycol, le dipropylèneglycol, le tripropylèneglycol, le tétrapropylèneglycol, le dibutylèneglycol, le tributylèneglycol ou le tétrabutylèneglycol ; à titre d'exemple, on peut plus particulièrement citer les Fomrez^{®} commercialisés par la société Witco et les poly(éthylène) adipate de la société Scientific Polymer Products,
- les polyesters polyol obtenus par polycondensation d'un acide dicarboxylique aliphatique avec au moins deux alcane-diols ou avec au moins un alcane-diol et au moins un hydroxyalkyl alcane-diol avec l'acide dicarboxylique aliphatique pouvant être l'acide adipique (acide hexane-1,6-dioïque) ; de tels polymères sont notamment décrits dans le document FR 2 836 381 (ou US 2003-224060),
- les polymères siliconés de polysilsesquioxane, notamment un polyalkylsilsesquioxane de formule : (R-SiO_{3/2})ₓₒ dans laquelle R représente un radical hydrocarboné, saturé ou insaturé, linéaire, ramifié ou cyclique; par exemple du type
- CₙH₂ₙ₊₁, avec n étant un entier allant de 1 à 20, notamment un radical méthyle, éthyle, propyle, butyle, pentyle, hexyle, heptyle, octyle, nonyle, décyle, dodécyle, tridécyle, tétradécyle, hexadécyle, octadécyle et eicosyle; ou encore un groupe aryle, notamment phényle ou tolyle; un groupe cycloalkyle, notamment cyclobutyle, cyclopentyle ou cyclohexyle ; un groupe alcényle, notamment vinyle ou allyle ; un groupe aralkyle, notamment 2-phényléthyle ou benzyle; R pouvant également comprendre un ou plusieurs atomes d'halogène, notamment de fluor ou de chlore ; de préférence R est un radical méthyle, éthyle, propyle ou phényle ; et x est le nombre de motifs, et peut être compris entre 1 et 10, notamment 1 à 4 ; à titre d'exemple, on peut citer la Belsil PMS MK^{®} de Wacker ou la Resin KR 220^{®} de Shin Etsu,
- les polyesters dendritiques à fonction hydroxyle terminale notamment ceux décrits dans le document FR 2 790 405 (ou US 6,379,683),
- les polymères hydrodipersibles mais néanmoins solubles dans des solvants non miscibles à l'eau comme par exemple : les polyesters, poly(ester amides), polyuréthannes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leur mélanges.

Lorsqu'ils sont présents, les polymères possédant un poids moléculaire en poids supérieur ou égal à 10 000 sont mis en oeuvre en une teneur au plus égale à 20 % en poids par rapport au poids total de la composition, en particulier de 1 à 15 %, et plus particulièrement de 1 à 10 % en poids par rapport au poids total de la composition.

### Phase huileuse

On entend désigner par "phase huileuse", au sens de la présente invention, une phase comprenant au moins une huile.

Une phase huileuse selon l'invention peut comprendre un agent actif liposoluble ou lipodispersible ou peut former elle-même un agent actif. Ainsi, le cas échéant une phase huileuse convenant à l'invention permet la solubilisation des agents actifs liposolubles et/ou la dispersion des agents actifs lipodispersibles.

Selon un mode de réalisation, une phase huileuse convenant à l'invention peut comprendre une huile possédant une forte polarité, à l'image de, par exemple, la N-lauroyle sarcosinate d'isopropyl (Eldew SL 205) de chez Ajinomoto afin de conférer au mélange de l'invention une température d'homogénéité T_{H} telle que définie ci-dessus.

Selon un autre mode de réalisation, une phase huileuse convenant à l'invention peut comprendre une huile possédant une faible polarité à l'image de, par exemple, d'un alcane tel que le polyisobutène hydrogéné (commercialisé sous la marque Parléam^{®} par la société NOF) ou l'isoparaffine 6-8 mole hydrogénée, afin de conférer au mélange de l'invention une température de coacervation T_{c} telle que définie ci-dessus.

Une phase huileuse convenant à l'invention peut comprendre au moins une huile choisie parmi des huiles non volatiles, et leurs mélanges.

Les huiles convenant à la mise en oeuvre de l'invention peuvent être choisies parmi des huiles végétales, des huiles animales, des huiles minérales, ou des huiles synthétiques, et leurs mélanges.

Elles peuvent être de type hydrocarboné telles que, par exemple, les triglycérides, esters, alcanes, polyoléfines, de type siliconée ou de type fluorée, modifiée ou non.

On entend désigner par "huile hydrocarbonée", une huile contenant principalement des atomes d'hydrogène et de carbone et éventuellement des atomes d'oxygène, d'azote, de soufre et/ou de phosphore.

Au sens de la présente invention, on entend par "huile fluorée", une huile comprenant au moins un atome de fluor.

Au sens de la présente invention, on entend par "huile siliconée", une huile comprenant au moins un atome de silicium, et notamment au moins un groupe Si-O.

Selon un mode de réalisation, elles peuvent être utilisées seules ou en mélange, entre elles ou avec d'autres composés tels que définis, par exemple, par la suite.

Les huiles non volatiles peuvent, notamment, être choisies parmi les huiles hydrocarbonées, le cas échéant fluorées et/ou les huiles siliconées non volatiles.

Comme huile hydrocarbonée non volatile, on peut notamment citer :
- les huiles hydrocarbonées d'origine animale, telle que le squalane ;
- les huiles hydrocarbonées d'origine végétale telles que les esters de phytostéaryle, comme l'oléate de phytostéaryle, l'isostéarate de phystostéaryle et le glutamate de lauroyl/octyldodécyle/phytostéaryle (AJINOMOTO, ELDEW PS203) ; les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées, ces huiles sont notamment des triglycérides héptanoïques ou octanoïques ; les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810^{®}, 812^{®} et 818^{®} par la société DYNAMIT NOBEL et leurs mélanges,
- les hydrocarbures linéaires ou ramifiés, d'origine minérale ou synthétique tels que la vaseline, les polydécènes, le polyisobutène hydrogéné tel que le parléam, et leurs mélanges ;
- les éthers de synthèse ayant de 10 à 40 atomes de carbone ;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10 ;
- et leurs mélanges.

Les esters peuvent être notamment choisis parmi les esters d'acide gras comme par exemple :
- l'octanoate de cétostéaryle, les esters de l'alcool isopropylique, tels que le myristate d'isopropyle, le palmitate d'isopropyle, le lauroylsarcosinate d'isopropyle (ELDEW SL 205, d'Ajinomoto), le palmitate d'éthyle, le palmitate de 2-éthyl-hexyle, le stéarate ou l'isostéarate d'isopropyle, l'isostéarate d'isostéaryle, le stéarate d'isocétyle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, les octanoates, décanoates ou ricinoléates d'alcools ou de polyalcools comme le dioctanoate de propylène glycol, l'octanoate de cétyle, l'octanoate de tridécyle, le 4-diheptanoate d'éthyle 2-hexyle, le diheptanoate de polyéthylène glycol, le diétyl 2-d'hexanoate de propylèneglycol et leurs mélanges, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle, le benzoate d'alkyle, les benzoates d'alcools en C₁₂ à C₁₅, et leurs mélanges ;
- les esters de polyols et les esters du pentaérythritol, comme le tétrahydroxystéarate/tétraisostéarate de dipentaérythritol ;
- les esters de dimères diols et dimères diacides tels que les Lusplan DD-DA5^{®} et DD-DA7^{®}, et leurs mélanges, commercialisés par la société NIPPON FINE CHEMICAL et décrits dans la demande FR 2851 915 (ou US 2004-175338) déposée le 6 mars 2003;
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol ;
- les acides gras supérieurs liquides tels que l'acide oléique, l'acide linoléique, l'acide linolénique, et leurs mélanges ;
- les carbonates de di-alkyle, les 2 chaînes alkyles pouvant être identiques ou différentes, tel que le dicaprylyl carbonate commercialisé sous la dénomination CETIOL CC^{®}, par COGNIS ; et
- leurs mélanges.

Les huiles de silicone non volatiles utilisables dans la composition selon l'invention peuvent être les polydiméthylsiloxanes (PDMS) non volatiles, comme la siméthicone, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, et les 2-phényléthyltriméthylsiloxysilicates, les diméthicones ou phényltriméthicone de viscosité inférieure ou égale à 100 cSt, et leurs mélanges.

Une phase huileuse convenant à l'invention peut éventuellement comprendre au moins une huile volatile, le cas échéant choisie parmi les huiles hydrocarbonées volatiles, les silicones volatiles, les huiles volatiles fluorées, et leurs mélanges.

Selon un mode de réalisation, une phase huileuse convenant à l'invention peut comprendre au moins une huile choisie parmi les huiles hydrocarbonées d'origine animale, les huiles hydrocarbonées d'origine végétale, les hydrocarbures linéaires ou ramifiés, d'origine minérale ou synthétique, les éthers de synthèse ayant de 10 à 40 atomes de carbone, les esters de synthèse de formule R₁COOR₂ dans laquelle R₁ représente le reste d'un acide gras linéaire ou ramifié comportant de 1 à 40 atomes de carbone et R₂ représente une chaine hydrocarbonée notamment ramifiée contenant de 1 à 40 atomes de carbone à condition que R₁ + R₂ soit > 10, les huiles de silicone non-volatiles, et leurs mélanges.

Selon une variante de réalisation, une phase huileuse convenant à l'invention peut comprendre au moins une huile choisie parmi les triglycérides d'acide caprique/caprylique, le stéarate d'isocétyle, la N-lauroyle sarcosinate d'isopropyle, et leurs mélanges.

Selon un mode de réalisation, une phase huileuse de type N-lauroyle sarcosinate d'isopropyle peut avantageusement être mise en oeuvre avec des oligomères choisis parmi les polycaprolactones, les polyesters de polyol(s), les poly(alkylène adipate) et des mélanges de ceux-ci.

Un tel mélange peut être avantageusement mis en oeuvre pour l'encapsulation de Gatuline dermasensitive^{®} (extrait de bourgeons de caprier dans l'octyldodécyl myristate) de chez Gattefosse, des fractions liquides et/ou solides du karité, des Nutralipids^{®} HY (mélange d'huile de passiflore, d'amande d'abricot, de maïs, et de son de riz) de chez Nestlé World Trade Corporation, et/ou des huiles de rosiers muscats.

Selon un mode de réalisation, une phase huileuse de type triglycérides d'acides caprique/caprylique peut avantageusement être mise en oeuvre avec des oligomères choisis parmi les polycaprolactones et des mélanges de ceux-ci.

Un tel mélange peut avantageusement être mis en oeuvre pour l'encapsulation d'agents actifs choisis parmi des extraits de gingembre, de l'acétate de vitamine E, et des mélanges de ceux-ci.

Selon un mode de réalisation, une phase huileuse de type stéarate d'isocétyle peut avantageusement être mise en oeuvre avec des oligomères choisis parmi les polyesters de polyol(s) et des mélanges de ceux-ci.

Selon un autre mode de réalisation une phase huileuse comprenant un mélange d'huile choisie parmi :
- la N-lauroyle sarcosinate d'isopropyle et les triglycérides d'acide caprique/caprylique, ou
- la N-lauroyle sarcosinate d'isopropyle et le stéarate d'isocétyle, peut avantageusement être mise en oeuvre avec des oligomères choisis parmi des polyesters de polyol(s), des poly(alkylène adipate) et des mélanges de ceux-ci.

Selon un mode de réalisation, une phase huileuse convenant à l'invention peut comprendre un agent actif liposoluble et/ou un agent actif lipodispersible et/ou être formée d'un agent actif liposoluble huileux tel que défini ci-après.

### Agents actifs

Un agent actif convenant à l'invention peut être formé par la phase huileuse, ou solubilisé ou dispersé dans une phase huileuse de l'invention.

A titre d'agent actif convenant à l'invention, on peut citer les agents actifs liposolubles choisis parmi les substances huileuses, liquides à partir de 40 °C, naturelles, végétales ou animales, ou synthétiques ayant ou non une ou plusieurs activités biologiques avérées et insolubles dans l'eau (moins de 2 % en poids à température ambiante), les huiles végétales riches en insaturations comme l'huile de bourrache, les huiles de poisson, les filtres solaires, les vitamines E, F, K, leurs esters, et leurs mélanges, des vitamines telles que la vitamine A (rétinol), la vitamine D, les carotènes tel que le β-carotène, l'acide salicylique, des extraits de gingembre, l'huile de rosier muscat, les céramides, l'acide alpha-linoléïque, de la Gatuline dermasensitive^{®} (extrait de bourgeons de caprier dans l'octyldodécyl myristate), des fractions liquides et solides de karité, les Nutralipids^{®} HY (mélange d'huile de passiflore, d'amande d'abricot, de maïs, et de son de riz), des dérivés de ceux-ci, et des mélanges de ceux-ci. Par exemple, peuvent être utilisés à titre de dérivés de l'acide salicylique ceux décrits dans les documents FR-A-2 581 542 (ou US 4,767,750), EP-A-378 936 (US 5,262,407) et EP-A-570230 (ou US 5,580,549) et en particulier les acides n-octanoyl-5-salicylique, n-décanoyl-5-salicylique, n-dodécanoyl-5-salicylique, noctyl-5-salicylique, n-heptyloxy-5-salicylique et n-heptyloxy-4-salicylique.

A titre d'agent actif convenant en particulier à l'invention on peut mentionner la Gatuline dermasensitivé^{®} (extrait de bourgeons de caprier dans l'octyldodécyl myristate) de chez Gattefosse, des extraits de gingembre, des acétates de vitamines, notamment de vitamine E, des fractions liquides et solides de karité, l'huile de rosier muscat, les Nutralipids^{®} HY (mélange d'huile de passiflore, d'amande d'abricot, de maïs, et de son de riz) de chez Nestlé, des dérivés de ceux-ci et des mélanges de ceux-ci.

A titre d'exemples d'agents actifs lipodispersibles convenant à l'invention, on peut mentionner, l'acide ellagique, l'acide glycyrrhétinique, et leurs mélanges.

Selon un mode de réalisation, une phase huileuse de l'invention peut en outre comprendre des agents colorants liposolubles ou lipodispersibles tels que des pigments ou des nacres.

Par ailleurs, peuvent également être encapsulées, des agents actifs à effet thérapeutique utilisées dans le domaine pharmaceutique dans la mesure où ils présentent un caractère huileux et/ou une solubilité et/ou une dispersibilité suffisante dans la phase huileuse considérée. Il peut s'agir d'anti-inflammatoires stéroïdiens ou non, d'antifongiques, d'antibactériens, d'antibiotiques, d'antimitotiques, d'anesthésiques, d'analgésiques, d'antiseptiques, d'antiviraux, voire de mélanges de ceux-ci.

### Agents tensioactifs

Selon un mode de réalisation, un procédé selon l'invention peut mettre en oeuvre au moins un agent tensioactif.

Le(s) agent(s) tensioactif(s) peu(ven)t être mis en oeuvre en phase huileuse et/ou en phase aqueuse.

Un agent tensioactif convenant à l'invention peut être choisi parmi les agents tensioactifs ioniques, anioniques, cationiques, non-ioniques, et leurs mélanges.

Afin de faciliter l'émulsification de la phase huileuse et le cas échéant de l'agent actif à encapsuler, et de maîtriser la stabilité de l'émulsion correspondante, il peut être souhaitable d'utiliser au moins un agent tensioactif, notamment un agent tensioactif non ionique.

Un agent tensioactif ou un mélange de tensioactifs peut être présent de 0,05 à 25 % et notamment de 1 à 20 % en poids par rapport au poids de la phase huileuse à disperser. Sa valeur HLB (Balance Hydrophile-Lipophile) peut être ajustée pour être favorable à la formation des émulsions de type huile dans eau.

Conviennent par exemple à l'invention, les agents tensioactifs non ioniques suivants :
- les alkylester ou éther de glycérol ou de polyglygérol constitués de 1 à 10 « motif(s) » glycérol et d'au moins une chaîne alkyle (acide pour les esters et alcool pour les éthers) ayant de 12 à 22 atomes de carbone. Elle peut être saturée ou insaturée et ramifiée ou non. A titre d'exemple, on peut citer le Nikkol DGMS^{®} (monostéarate de diglycérol), le Nikkol decaglyn 21S^{®} (di-isostéarate de décaglycérol), l'hexadécyléther de triglycérol,
- les esters mixtes d'acides gras ou d'alcool gras, d'acide carboxylique et de glycéryl choisis par exemple parmi les esters mixtes d'acide gras ou d'alcool gras enC₈-C₂₂ et d'alpha-hydroxyacide et/ou d'acide succinique avec la glycérine et leurs mélanges. A titre d'exemple, on peut citer l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375^{®} ; l'ester mixte d'acide succinique et d'alcool isostéarylique avec la glycérine (nom CTFA : Isostéaryl diglycéryl succinate) commercialisé par la société Hüls sous la dénomination Imwitor 780 K^{®} ; l'ester mixte d'acide citrique et d'acide stéarique avec la glycérine (nom CTFA : Glycéryl stéarate citrate) commercialisé par la société Hüls sous la dénomination Imwitor 370^{®} ; l'ester mixte d'acide lactique et d'acide stéarique avec la glycérine (nom CTFA : Glyceryl stearate lactate) commercialisé par la société Danisco sous la dénomination Lactodan B30® ou Rylo LA30^{®},
- les éthers gras éthoxylés ou esters gras éthoxylés comprenant de 2 à 50 unités d'oxyde d'éthylène et au moins une chaîne alkyle (acide pour les esters et alcool pour les éthers) ayant de 12 à 22 atomes de carbone. Elle peut être saturée ou insaturée et ramifiée ou non. A titre d'exemple, on citera la série des Brij^{®} (alcools gras éthoxylés) commercialisée par la société Uniqema, la série des Myrj^{®} (stéarates éthoxylés) commercialisée par la société Uniqema et l'isostéarate de PEG400 également commercialisé par Uniqema,
- les esters gras de sorbitan, oxyéthylénés ou non. Ils comprennent au moins un motif sorbitan et dans le cas où ils sont oxyéthylénés, de 2 à 50 motifs d'oxyde d'éthylène, et au moins une chaîne alkyle (acide gras) ayant de 12 à 22 atomes de carbone. Elle peut être saturée ou insaturée et ramifiée ou non. A titre d'exemple, on citera les séries des Span^{®} (esters de sorbitan) et des Tween^{®} (esters de sorbitan oxyéthylénés) commercialisées par Uniqema,
- les esters gras de sucre ou éthers gras de sucre. Le tensioactif utilisé est par exemple choisi parmi les esters d'acide gras en C₈ à C₂₂ de sucrose, maltose, glucose et fructose, les esters d'acide gras en C₁₄ à C₂₂ et de méthylglucose, les alkylpolyglucosides et leurs mélanges. La ou les chaînes alkyles peuvent être saturée ou insaturée et ramifiée ou non.

Les acides gras en C₈-C₂₂ ou en C₁₄-C₂₂ formant le motif gras des esters utilisables dans l'invention comportent au moins une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On peut utiliser des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta^{®} F50, F70, F110, F160 ayant respectivement un HLB (Balance Hydrophile Lipophile) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450^{®}. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme tensioactifs dans la composition selon l'invention peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables selon l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle et leurs mélanges tels que cétéaryle.

A titre d'exemple d'éthers d'alcool gras et de sucre, on peut citer les alkylpolyglucosides tels que le décyl glucoside et le lauryl glucoside commercialisés par exemple par la société Henkel sous les dénominations respectives Plantaren 2000^{®} et Plantaren 1200^{®}, le cétostéaryl glucoside éventuellement en mélange avec l'alcool cétostéarylique, commercialisé par exemple sous la dénomination Montanov 68^{®} par la société Seppic, sous la dénomination Tego-care CG90^{®} par la société Goldschmidt et sous la dénomination Emulgade KE3302^{®} par la société Henkel, ainsi que l'arachidyl glucoside, par exemple sous la forme du mélange d'alcools arachidique et béhénique et d'arachidyl glucoside commercialisé sous la dénomination Montanov 202^{®} par la société Seppic,
- les copolymères blocs d'oxyde d'éthylène et d'oxyde de propylène. Les copolymères blocs d'oxyde d'éthylène et d'oxyde de propylène, utilisables comme tensioactifs dans les compositions selon l'invention peuvent être choisis notamment parmi les copolymères blocs de formule (A) :

   HO(C₂H₄O)ₓ(C₃H₆O)_{y}(C₂H₄O)_{z}H (A)

   dans laquelle x, y et z sont des nombres entiers tels que x+z peut aller de 2 à 280 et peut aller de 14 à 100. Ces polymères sont notamment commercialisés sous le nom de Pluronic^{®} ou Lutrol^{®} par BASF, Symperonic^{®} par Uniqema,
- les lécithines de soja ou d'oeuf hydrogénée ou non, enrichies en phosphatidylcholine ou non,
- les tensioactifs siliconé comportant au moins une chaîne oxyéthylénée et ou oxypropylénée. On peut citer, à titre d'exemple, ceux décrits dans les brevets US A 5,364,633 et US A 5,411,744 et par exemple un composé de formule (I) : 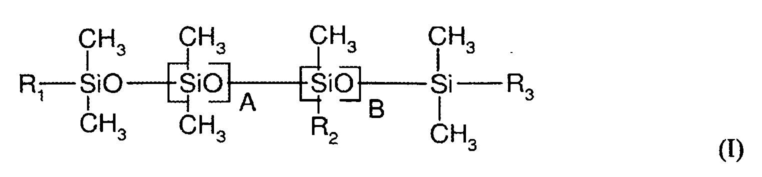 dans laquelle :
   R₁, R₂, R₃, indépendamment les uns des autres, représentent un radical alkyle en C₁-C₆ ou un radical -(CH₂)ₓ-(OCH₂CH₂)y-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.

Selon un mode de réalisation particulier, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 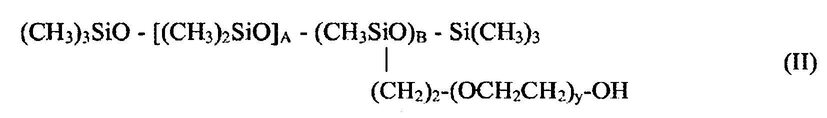 dans laquelle A est un nombre entier allant de 20 à 105, B est un nombre entier allant de 2 à 10 et y est un nombre entier allant de 10 à 20. On peut également citer à titre d'exemple de tensioactifs siliconés de formule (I), les composés de formule (III) :

HO-(CH_{2C}H₂O)_{y}-(CH₂)₃-[(CH₃)₂SiO]_{A}'-(CH₂)₃-(OCH₂CH₂)_{y}-OH (III)

dans laquelle A' et y sont des nombres entiers allant de 10 à 20.

On peut utiliser comme composés ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12. Le composé Q4-3667 est un composé de formule (III) où A est 15 et y est 13. Mais aussi :
- les citrates d'alkyléther,
- les alkényl succinates alkoxylés,
- les alkényl succinates de glucose alkoxylés,
- les alkényl succinates de méthylglucose alkoxylés.

Ces tensioactifs peuvent être utilisés seuls ou en association. Leur taux, par rapport à la phase huileuse encapsulée peut être compris entre 0,1 et 30 % en poids.

Afin d'améliorer la stabilité de l'émulsion, voire de réduire un peu plus la taille des gouttes, il peut être possible d'ajouter de 0,01 à 5 % en poids par rapport au poids total de la dispersion d'au moins un tensioactif ionique, soluble dans l'eau et ayant une HLB supérieure à 11. Ce type de tensioactif ionique génère semble-t-il une charge électrique à la surface des nanocapsules et favorise ainsi la manifestation de répulsions électrostatiques entres elles. Ce ou ces tensioactifs ioniques, anioniques ou cationiques peuvent être choisis parmi :
- les lipides amphiphiles anioniques comme :

- les sels alcalins du dicétyl- et du dimyristyl-phosphate,
- les sels alcalins du cholestérol sulfate,
- les sels alcalins du cholestérol phosphate,
- les lipoaminoacides et leurs sels tels que les acylglutamates mono- et disodiques comme le sel disodique de l'acide N-stéaroyl L-glutamique commercialisé sous la dénomination Amisoft HS21P par la société AJINOMOTO,
- les sels de sodium de l'acide phosphatidique,
- les phospholipides,
- les dérivés alkylsulfoniques notamment de formule : 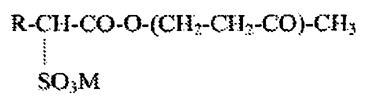 dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium,
- les lipides amphiphiles cationiques de type sels d'ammonium quaternaire, amines grasses et leurs sels comme par exemple :
- les sels d'ammonium quaternaires de formule générale (IV) suivante : 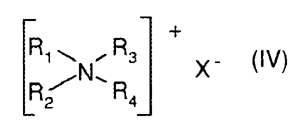 dans laquelle les radicaux R₁, R₂, R₃ et R₄ peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl (C₂-C₆)sulfates, alkyl-ou-alkylarylsulfonates. Comme sels d'ammonium quaternaire de formule (IV), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (acétate de myristyle) ammonium vendu sous la dénomination «CERAPHYL 70®» par la société VAN DYK,
- les sels d'ammonium quaternaire de l'imidazolinium, comme par exemple ceux de formule (V) suivante : 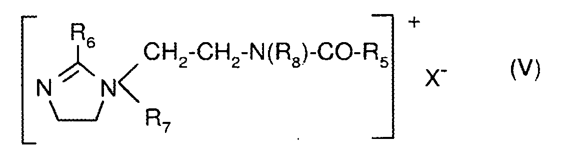 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R8 représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. R₅ et R₆ peuvent désigner un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination «REWOQUAT W 75» par la société REWO,
- les sels de diammonium quaternaire de formule (VI) suivante : 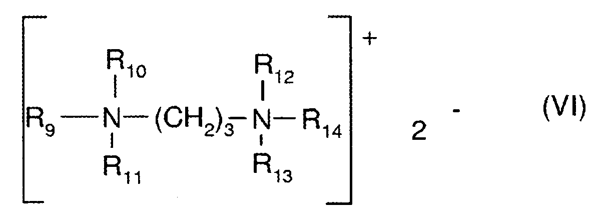 dans laquelle R₉ désigne un radical aliphatique comportant environ de 16 à 30 atomes de carbone ; R₁₀, R₁₁, R₁₂, R₁₃, et R₁₄ sont choisis parmi l'hydrogène ou un radical alkyle comportant de 1 à 4 atomes de carbone; et X est un anion choisi dans le groupe des halogénures, acétates, phosphates, nitrates et méthylsulfates. De tels sels de diammonium quaternaire comprennent notamment le dichlorure de propane suif diammonium.

Le taux de tensioactif ionique lorsqu'il est associé au(x) tensioactif(s) non ionique(s) présent(s) dans le milieu aqueux peut être ajusté de manière à représenter de 2 à 100 % en poids du poids de celui-ci.

### Composés aptes à former une phase lamellaire

Il est souvent souhaitable ou nécessaire de pourvoir les capsules de l'invention d'un enrobage dit « lamellaire ». Il s'agit d'une structure organisée en un ou plusieurs feuillet(s) lipidique(s) constitué(s) chacun d'une bicouche de molécules amphiphiles semblable à celle des membranes biologiques.

L'enveloppe polymérique des capsules selon l'invention peut être ainsi entourée d'un enrobage lamellaire ayant une structure organisée en un ou plusieurs feuillet(s) constitué(s) chacun d'une double couche de molécules amphiphiles constituant un agent d'enrobage. Cet enrobage, outre sa fonction d'ajustement de la taille des capsules, améliore l'étanchéité des capsules vis-à-vis d'une fuite de l'actif vers une autre phase lipidique de la composition.

On entend par « phase lamellaire » (phase D selon EKWALL), une phase cristal liquide à symétrie plane, comprenant plusieurs bicouches d'amphiphile disposées en parallèle et séparées par un milieu liquide qui est généralement de l'eau.

Une définition plus précise de cette dénomination est donnée dans Ekwall (1968), Adv. Liq. Cryst. (Brown G.H., Ed.), Chap. 1, 14. Cette phase possède une texture caractéristique au microscope en lumière polarisée dont on pourra trouver une description plus précise dans Roservear (1968), JSCC, 19, 581. et dans Lachampt et Vila (1969), Revue Française des Corps Gras, n° 2, 87-111.

L'enrobage lamellaire est obtenu avec des agents tensioactifs à caractère hydrophobe, solubles dans la phase huileuse utilisée dans le procédé décrit ci-dessus et qui sont capables, en présence d'eau, de former les doubles couches lipidiques décrites ci-dessus. Dans le procédé d'encapsulation, utilisé par les inventeurs, le tensioactif d'enrobage peut être dissous dans la phase huileuse contenant les oligomères et le cas échéant le(s) agent(s) actif(s).

On peut citer à titre d'exemple de tels tensioactifs d'enrobage, les phospholipides tels que la lécithine comme décrit dans le document EP-A-447 318 (ou US 6,203,802) ; certains polycondensats d'oxyde d'éthylène et d'oxyde de propylène, comme les produits vendus sous la dénomination PLURONIC^{®} par la société BASF tels que PLURONIC^{®} L121 ou sous la dénomination SYNPERONIC^{®} par la société ICI ; ou les agents tensioactifs siliconés (silicones comportant au moins une chaîne oxyéthylénée et/ou oxypropylénée) capables de former des structures lamellaires, tels que ceux décrits dans les documents US-A-5,364,633 et US-A-5,411,744 utilisés dans la demande de brevet FR-A-2,742,677 (ou US 5,919,487), par exemple ceux vendus par la société DOW CORNING sous les dénominations DC 5329, DC 7439-146, DC 2-5695 et Q4-3667 ; et leurs mélanges.

### Agents réticulants

Selon un mode de réalisation, un procédé conforme à l'invention peut comprendre une étape g) de réticulation. Cette étape de réticulation peut être effectuée en présence d'au moins un agent réticulant.

Une étape de réticulation est avantageusement effectuée lorsque la température de fusion P_{f} du ou des polymères, en particuliers des oligomères, mis en oeuvre est inférieure à la température ambiante T_{amb}.

A titre d'exemples d'agents réticulants, convenant à l'invention, on peut citer les molécules di-fonctionnelles au minimum ou poly-fonctionnelles.

Le(s) agent(s) réticulant(s) peu(ven)t être de nature hydrophile pour une incorporation en phase aqueuse.

Le(s) agent(s) réticulant(s) peu(ven)t être de nature lipophile pour une incorporation en phase huileuse.

En particulier pour éviter une réaction délétère entre le(s) agent(s) actif(s) encapsulé(s) et l'agent réticulant, ce dernier sera en particulier choisi parmi les agent(s) réticulant(s) hydrophile(s).

Un tel agent sera en particulier ajouté en fin de procédé via la phase aqueuse.

A titre d'exemples de fonctions identiques ou différentes portées un agent réticulant convenant à l'invention, on peut citer l'isocyanate, l'anhydride mixte et le chlorure d'acide.

Un agent réticulant convenant à l'invention peut notamment être choisi parmi les polyisocyanates.

Les polyisocyanates peuvent être des oligomères de l'hexaméthylène diisocyanate (HDI) (tels que les RHODOCOAT® de chez RHODIA et les BASONAT® de chez BASF), du toluène diisocyanate (TDI) tel que le Vibrathane 6060 de chez Uniroyal Chemical, du méthylène bisphényl isocyanate (MDI) tel que les Vibrathane 8030 et 8045 ou de l'isophorone (IPDI) comme les HYPOL de chez DOW CHEMICALS.

Les agents réticulants hydrophiles peuvent être choisis parmi le RHODOCOAT WT2102 de chez RHODIA, le BASONAT HW100 de chez BAYER, le BAYHYDUR de chez BAYER ou l'HYPOL JT de chez DOW CHEMICALS.

Les agents réticulants lipophiles peuvent être choisis parmi le RHODOCOAT HDT-LV de chez RHODIA ou DESMODUR de chez BAYER

### Composition cosmétique, dermatologique ou pharmaceutique

Les capsules de l'invention peuvent être introduites dans tout type de formulation galénique, tel que les gels, les émulsions huile/eau, eau/huile, multiple (E/H/E, H/E/H), les sérums, les lotions, etc.

Selon un mode de réalisation, une suspension de capsules de l'invention peut être mise en oeuvre dans une composition en une teneur variant de 0,5 à 60 % en poids par rapport au poids total de la composition, en particulier variant de 0,5 à 40 % et plus particulièrement variant de 2 à 10 % en poids par rapport au poids total de la composition.

Une suspension de capsules de l'invention peut être utilisée pour la préparation d'une composition cosmétique, dermatologique ou pharmaceutique.

De telles compositions peuvent en particulier êtres destinées à véhiculer, notamment de façon améliorée, des actifs cosmétiques ou thérapeutiques. Ainsi, ces compositions peuvent conférer à ces actifs une biodisponibilité améliorée.

Ainsi, un objet de l'invention est une composition cosmétique, dermatologique ou pharmaceutique comprenant dans un milieu physiologiquement acceptable au moins une suspension aqueuse de capsules susceptible d'être obtenue selon un procédé de l'invention.

Les formulations contenant des capsules peuvent par exemple être destinées au soin et/ou au maquillage des cheveux, de la peau, des ongles.

Par ailleurs, les capsules de la présente invention peuvent être utilisées à des fins pharmaceutiques pour la vectorisation de médicaments, par voie orale, péritonéale, intraveineuse, intramusculaire ou ophtalmique.

Egalement, les capsules de l'invention peuvent être utilisées pour la préparation de compositions cosmétiques pouvant se présenter sous forme de mascara, de produit pour les sourcils, d'eyeliner, de fard à paupières, de fard à joues, de fond de teint, de produit pour les lèvres, de produit de maquillage du corps, de produit de maquillage ou de soin des cheveux.

Les compositions cosmétiques, dermatologiques ou pharmaceutiques de l'invention peuvent comprendre tout additif usuellement utilisé dans les domaines concernés, sous réserve que ces additifs n'altèrent pas la propriété des compositions, ni des capsules de l'invention.

Les exemples, donnés ci-après à titre purement illustratif et non limitatif, permettront de mieux comprendre l'invention.

Les quantités y sont indiquées en pourcentage en poids sauf indication contraire.

### EXEMPLE 1

### Capsules de beurre de karité

### Phase organique :

| | |
|---|---|
| Oligomère de polycaprolactone CAPA®2200A de chez SOLVAY | 2 g |
| Beurre de karité (Lipex 202 de chez Karlshamns) | 7 g |
| N-lauroyle sarcosinate d'isopropyle (Eldew SL-205 de chez Ajinomoto) | 8 g |

| Phase aqueuse : | |
|---|---|
| Acide N Stéaroyl L glutamique disodique (Amisoft HS21P d'Ajinomoto) | 0,5 g |
| Eau distillée | QSP 100 g |

Les phases huileuse et aqueuse sont chauffées à 80 °C. Une pré-émulsion organique/eau est réalisée à l'aide d'un agitateur Ultra Turrax (Ika) pendant 5 min à 10000 rpm dans un bain thermostaté à 80 °C. Cette pré-émulsion est ensuite homogénéisée, à 80 °C, à l'aide d'un homogénéisateur haute pression de type OBL20 de chez Niro Soavi, 2 fois à 600 bars.

Ensuite, l'émulsion est refroidie à la température ambiante par simple agitation au barreau aimanté.

Une suspension de capsules contenant 7 % de beurre de karité et 2 % d'oligomère en poids est obtenue.

Le contrôle de la taille des capsules est effectué à l'aide d'un granulomètre laser par diffusion de la lumière comme indiqué précédemment. La taille moyenne des capsules est de 170 nm.

Les capsules obtenues possèdent un indice de polydispersité, mesuré comme indiqué précédemment, de 0,183 .

Ces capsules sont parfaitement stables 2 mois à +4°C, à température ambiante et à 45 °C.

### EXEMPLE 2

### Post-réticulation des capsules de karité

Les capsules de karité sont préparées suivant l'exemple 1.

| | |
|---|---|
| capsules de karité | 100 g |
| Bayhydur 3100 (Bayer) | 0,5 g |

On ajoute 0,5 g de Bayhydur 3100 à 100g de suspension de capsules sous agitation à température ambiante et sous hotte.

### EXEMPLES 3-19

Des capsules avec (exemples 3-11) ou sans (exemples 12-17) agent actif huileux sont préparées comme indiqué dans l'exemple 1.

La formulation de ces compositions comprend également 0,5 % d'acyl Glutamate HS 21 de Ajinomoto et de l'eau distillée q.s.p. 100.

Les résultats obtenus sont résumés dans le tableau ci-dessous.

| **Ex.** | **Nom de l'actif et %** | **Huiles associées et %** | **Oligomères** | **-Formation de capsules** |
|---|---|---|---|---|
| | | | | **-Diamètre moyen** |
| | | | | **-Indice de polydispersité** |
| | Gatuline derma- | Eldew SL 205 | Capa® 2302A | OK |
| **3** | sensitive | 10,5% | 2 % | 172 nm |
| | 4,5 % | | | 0,113 |
| | Extrait de gingembre | Triglycérides d'acide | Capa® 2200A | OK |
| **4** | 3,75 % | caprique/caprylique | 2 % | 173 nm |
| | | 11,25 % | | 0,193 |
| | Extrait de gingembre | Triglycérides d'acide | Capa® 2302A | OK |
| **5** | 3,75 % | caprique/caprylique | 2 % | 161nm |
| | | 11,25 % | | 0,091 |
| | Acétate de vitamine E | Triglycérides d'acide | Capa®3050 | OK |
| **6** | 0,66 % | caprique/caprylique | 0,5% | 166 nm |
| | | 12,6 % | | 0,137 |
| | Acétate de vitamine E | Triglycérides d'acide | Capa® 2200A | OK |
| **7** | 18,25% | caprique/caprylique | 2,5 % | 320 nm |
| | | 0,75 % | | 0,250 |
| | Acétate de vitamine E | Triglycérides d'acide | Capa® 2200A 2,5 % | OK |
| **8** | 18,25 % | caprique/caprylique | +Capa®3050 0,5 % | 286 nm |
| | | 0,75 % | | 0,255 |
| | Fraction liquide du karité | Eldew SL 205 | Capa® 2200A | OK |
| **9** | | 8% | 2 % | 160 nm |
| | 7 % | | | 0,183 |
| | Nutralipids HY | Eldew SL 205 | Capa® 2200A | OK |
| **10** | 7,5 % | 7,5 % | 2 % | 179 nm |
| | | | | 0,096 |
| | Huile de rosier muscat | Eldew SL 205 | Capa® 2200A | OK |
| **11** | 9,4 % | 5,6 % | 2 % | 172 nm |
| | | | | 0,146 |
| | Fraction liquide du karité | Eldew SL 205 | Capa® 2200A | OK |
| | | 8,4 % | 2 % | 192 nm |
| **12** | 3,3 % | | | 0,139 |
| | + fraction solide du karité | | | |
| | | | | |
| | 3,3 % | | | |
| | | Stéarate d'isocétyle | Eternacoll 3020 (UBE) | OK |
| **13** | | 8,6 % | 2 % | 180 nm |
| | | + Eldew SL 205 | | 0,140 |
| | | 6,4 % | | |
| | | Stéarate d'isocétyle | UH-Carb 50 (UBE) | OK |
| **14** | | 2% | 2% | 160 nm |
| | | + Triglycérides d'acide | | 0,187 |
| | | caprique/caprylique | | |
| | | 13 % | | |
| | | Triglycérides d'acide | UH-Carb 300 (UBE) 2 % | OK |
| **15** | | caprique/caprylique | | 192 nm |
| | | 10 % | | 0,150 |
| | | + Eldew SL205 | | |
| | | 5% | | |
| | | Triglycérides d'acide | Fomrez F930 (Crompton) | OK |
| **16** | | caprique/caprylique | 2 % | 165 nm |
| | | 5,6 % | | 0,157 |
| | | + Eldew SL205 | | |
| | | 9,4 % | | |
| | | Triglycérides d'acide | Lexorez 1151-35 (Inolex) | OK |
| **17** | | caprique/caprylique | 2 % | 167 nm |
| | | 3,75 % + Eldew SL205 | | 0,147 |
| | | 11,25 % | | |
| | | Triglycérides d'acide | Lexorez 1460-36 (Inolex) | OK |
| **18** | | caprique/caprylique | 2 % | 182 nm |
| | | 8,75 % | | 0,128 |
| | | + Eldew SL205 | | |
| | | 6,25 % | | |
| **19** | Fraction liquide du beurre de karité | Eldew SL 205 | Capa^{®} 2201 1 | OK |
| | | 11,2% | (Solvay) | 220 nm |
| | 4,4 % | | | 0,141 |
| | Fraction solide du beurre de karité | | | |
| | | | | |
| | 4,4 % | | | |

## Revendications

1. Procédé de préparation d'une suspension de capsules de type noyau/écorce comprenant au moins les étapes de :
a) disposer d'un mélange hétérogène à température ambiante (T_{amb}) comprenant :
- une phase huileuse,
- des polymères, identiques ou différents, non miscibles à l'eau et à la phase huileuse, dont au moins un polymère est un oligomère possédant un poids moléculaire en poids inférieur à 10 000, et un indice d'hydroxyle (I_{OH}) supérieur ou égal à 10 mg KOH/g,
b) homogénéiser la phase huileuse et les polymères par chauffage, à une température d'homogénéisation T_{H}, du mélange obtenue à l'étape a), T_{H} étant inférieure à 100 °C, ledit mélange possédant, à la température T_{H}, une viscosité inférieur ou égale à 17 mPa.s⁻¹,
c) disperser, à la température T_{H}, le mélange homogène obtenu à l'étape b) dans une phase aqueuse, pour obtenir une pré-émulsion directe dont la phase huileuse comprend les polymères,
d) soumettre la pré-émulsion obtenue à l'étape c) à un ensemble de forces de cisaillement appropriées à la réduction du diamètre des particules du mélange dispersé à une taille moyenne inférieure ou égale à environ 50 µm, et
e) refroidir l'émulsion obtenue à l'étape d) à une température T_{c} propice à la coacervation desdits polymères et à l'enrobage de gouttes de ladite phase huileuse par les coacervats,
f) refroidir la suspension obtenue à l'étape e) à une température Tₚ propice à la formation des capsules attendues par la précipitation et/ou la cristallisation des coacervats,
la phase huileuse étant propice à la formation d'un mélange homogène avec lesdits polymères, à une température T_{H}, et à la manifestation d'un phénomène de coacervation desdits polymères à une température T_{c}.

2. Procédé selon la revendication précédente, comprenant une étape g) de réticulation des polymères.

3. Procédé selon la revendication 1 ou 2 dans lequel T_{c} est supérieure ou égale à 45 °C.

4. Procédé selon l'une quelconque des revendications précédentes, dans lequel la suspension de capsules présente un taux en capsules variant de 3 à 90 %, en particulier variant de 10 à 60 % et plus particulièrement variant de 20 à 50 % en poids par rapport au poids total de la suspension.

5. Procédé selon l'une quelconque des revendications précédentes, dans lequel les polymères et la phase huileuse sont présents selon un rapport pondéral variant de 5/1 à 1/20, en particulier variant de 2/1 à 1/20, et plus particulièrement variant de 2/1 à 1/10.

6. Procédé selon l'une quelconque des revendications précédentes, dans lequel la phase huileuse comprend au moins un agent actif liposoluble et/ou un agent actif lipodispersible.

7. Procédé selon l'une quelconque des revendications précédentes, comprenant à l'étape a) des polymères de poids moléculaire supérieur ou égal à 10 000, en particulier supérieur ou égal à 15 000.

8. Procédé selon l'une quelconque des revendications précédentes, dans lequel lesdits polymères possèdent un point de fusion P_{f} inférieur à 100 °C, en particulier variant de -20 °C à 95 °C, et plus particulièrement variant de -10 °C à 80 °C.

9. Procédé selon l'une quelconque des revendications précédentes, dans lequel ledit ou lesdits oligomères sont choisis parmi les polycaprolactones, les poly-L-lactides, les poly-DL-lactides, les polyglycolides et leurs copolymères, les polymères de l'acide 3-hydroxybutyrique et ses copolymères avec l'acide l'hydroxyvalérique, les polycarbonates diols, les poly(alkylène adipate), les polyesters polyols, les polyesters dendritiques à fonction hydroxyle terminale, et leurs mélanges.

10. Procédé selon l'une quelconque des revendications précédentes, dans lequel la phase huileuse forme un agent actif.

11. Procédé selon l'une quelconque des revendications précédentes, dans lequel la phase huileuse comprend au moins une huile choisie parmi les huiles non volatiles, et des mélanges de celles-ci.

12. Procédé selon l'une quelconque des revendications précédentes, dans lequel est mis en oeuvre au moins un agent tensioactif en phase huileuse et/ou en phase aqueuse.

13. Procédé selon l'une quelconque des revendications 2 à 12, dans lequel l'étape g) de réticulation met en oeuvre au moins un agent réticulant, choisi parmi les molécules di-fonctionnelles ou poly-fonctionnelles, hydrophiles ou lipophiles.

14. Procédé selon la revendication précédente, dans lequel les fonctions de l'agent réticulant, identiques ou différentes, sont choisies parmi l'isocyanate, l'anhydre mixte, le chlorure d'acide.

15. Utilisation d'une suspension de capsules obtenue selon le procédé tel que défini selon l'une quelconque des revendications 1 à 14 pour la préparation d'une composition cosmétique, dermatologique ou pharmaceutique.

16. Composition cosmétique, dermatologique ou pharmaceutique comprenant, dans un milieu physiologiquement acceptable, une suspension de capsules susceptible d'être obtenue selon le procédé tel que défini selon l'une quelconque des revendications 1 à 14.

17. Capsules susceptibles d'être obtenues au moyen du procédé tel que défini selon l'une quelconque des revendications 1 à 14.

18. Suspension aqueuse de capsules susceptibles d'être obtenue au moyen du procédé tel que défini selon l'une quelconque des revendications 1 à 14.

## Claims

1. Process for the preparation of a suspension of capsules of core/shell type comprising at least the steps of:
a) having a heterogeneous mixture at ambient temperature (T_{amb}) comprising:
- an oily phase,
- identical or different polymers which are immiscible with water and with the oily phase, at least one polymer of which is an oligomer having a weight-average molecular weight of less than 10 000 and a hydroxyl number (N_{OH}) of greater than or equal to 10 mg KOH/g,
b) homogenizing the oily phase and the polymers by heating the mixture obtained in step a) at a homogenization temperature T_{H}, T_{H} being less than 100°C, the said mixture having, at the temperature T_{H}, a viscosity of less than or equal to 17 mPa·s,
c) dispersing, at the temperature T_{H}, the homogeneous mixture obtained in step b) in an aqueous phase, in order to obtain a direct preemulsion, the oily phase of which comprises the polymers,
d) subjecting the preemulsion obtained in step c) to a combination of shear forces appropriate for the reduction in the diameter of the particles of the dispersed mixture to a mean size of less than or equal to approximately 50 µm,
e) cooling the emulsion obtained in step d) to a temperature T_{c} suitable for the coacervation of the said polymers and for the coating of drops of the said oily phase by the coacervates, and
f) cooling the suspension obtained in step e) to a temperature Tₚ suitable for the formation of the expected capsules by the precipitation and/or crystallization of the coacervates,
the oily phase being suitable for the formation of a homogeneous mixture with the said polymers, at a temperature T_{H}, and for the appearance of a phenomenon of coacervation of the said polymers at a temperature T_{c}.

2. Process according to the preceding claim, comprising a step g) of crosslinking of the polymers.

3. Process according to claim 1 or 2, in which T_{c} is greater than or equal to 45°C.

4. Process according to anyone of the preceding claims, in which the suspension of capsules exhibits a level of capsules varying from 3 to 90% in particular varying from 10 to 60%, and more particularly varying from 20 to 50% by weight, with respect to the total weight of the suspension.

5. Process according to anyone of the preceding claims, in which the polymers and the oily phase are present according to a ratio by weight varying from 5/1 to 1/20, in particular varying from 2/1 to 1/20 and more particularly varying from 2/1 to 1/10.

6. Process according to anyone of the preceding claims, in which the oily phase comprises at least one fat-soluble active agent and/or one fat-dispersible active agent.

7. Process according to anyone of the preceding claims, comprising, in step a), polymers with a molecular weight of greater than or equal to 10 000, in particular greater than or equal to 15 000.

8. Process according to anyone of the preceding claims, in which the said polymers have a melting point Mp of less than 100°C, in particular varying from -20° C to 95° C and more particularly varying from -10° C to 80° C.

9. Process according to anyone of the preceding claims, in which the said oligomer(s) are selected from the group consisting in polycaprolactones, poly-L-lactides, poly-DL-lactides, polyglycolides arid their copolymers, polymers of 3-hydroxybutyric acid and its copolymers with hydroxyvaleric acid, polycarbonate diols, polyalkylene adipates, polyester polyols, dendritic polyesters comprising an end hydroxyl functional group, and their blends.

10. Process according to anyone of the preceding claims, in which the oily phase forms an active agent.

11. Process according to anyone of the preceding claims, in which the oily phase comprises at least one oil selected from the group consisting in nonvolatile oils, and mixtures of these.

12. Process according to anyone of the preceding claims, in which at least one surface-active agent is employed in the oily phase and/or in the aqueous phase.

13. Process according to anyone of claims 2 to 12, in which the crosslinking step g) employs at least one crosslinking agent selected from the group consisting in hydrophilic or lipophilic molecules which are difunctional or polyfunctional.

14. Process according to the preceding claim, in which the identical or different functional groups of the crosslinking agent are selected from the group consisting in isocyanate, mixed anhydride or acid chloride.

15. Use of a suspension of capsules which is obtained according to the process as defined according to anyone of claims 1 to 14 for the preparation of a cosmetic, dermatological or pharmaceutical composition.

16. Cosmetic, dermatological or pharmaceutical composition comprising, in a physiologically acceptable medium, a suspension of capsules which is capable of being obtained according to the process as defined according to anyone of claims 1 to 14.

17. Capsules capable of being obtained by means of the process as defined according to anyone of claims 1 to 14.

18. Aqueous suspension of capsules which is capable of being obtained by means of the process as defined according to anyone of claims 1 to 14.

## Patentansprüche

1. Verfahren zum Zubereiten einer Suspension von Kapseln des Kern/Schalen-Typs, das wenigstens die folgenden Schritte umfasst:
a) Bringen auf Umgebungstemperatur (T_{amb}) eines heterogenen Gemisches, das enthält:
- eine ölige Phase,
- Polymere, die gleich oder verschieden sind und mit Wasser und mit der öligen Phase nicht mischbar sind, wovon wenigstens ein Polymer ein Oligomer ist, das ein Molekulargewicht kleiner als 10000 besitzt und einen Hydroxyl-Index (I_{OH}) größer oder gleich 10 mg KOH/g besitzt,
b) Homogenisieren der öligen Phase und der Polymere durch Erwärmen auf eine Homogenisierungstemperatur (T_{H}) des im Schritt a) erhaltenen Gemisches, wobei T_{H} kleiner als 100 °C ist, wobei das Gemisch bei der Temperatur T_{H} eine Viskosität besitzt, die kleiner oder gleich 17 MPa · s⁻¹ ist,
c) Dispergieren des im Schritt b) erhaltenen homogenen Gemisches bei der Temperatur T_{H} in einer wässrigen Phase, um eine direkte Voremulsion zu erhalten, deren ölige Phase die Polymere enthält,
d) Beaufschlagen der im Schritt c) erhaltenen Voremulsion mit einer Gesamtheit von Scherungskräften, die für die Verkleinerung des Durchmessers der Partikel des dispergierten Gemisches auf eine mittlere Größe kleiner oder gleich etwa 50 µm geeignet sind, und
e) Abkühlen der im Schritt d) erhaltenen Emulsion auf eine Temperatur T_{c}, die für die Koazervierung der Polymere und für die Umhüllung der Tropfen der öligen Phase durch die Koazervate günstig ist,
f) Abkühlen der im Schritt e) erhaltenen Suspension auf eine Temperatur Tₚ, die für die Bildung der Kapseln, die durch Niederschlag und/oder Kristallisation der Koazervate erwartet werden, günstig ist,
wobei die ölige Phase für die Bildung eines homogenen Gemisches mit den Polymeren bei einer Temperatur T_{H} und für die Manifestation eines Koazervierungsphänomens der Polymere bei der Temperatur T_{c} geeignet ist.

2. Verfahren nach dem vorhergehenden Anspruch, das einen Schritt g) der Vernetzung der Polymere umfasst.

3. Verfahren nach Anspruch 1 oder 2, wobei T_{c} größer oder gleich 45 °C ist.

4. Verfahren nach einem der vorhergehenden Ansprüche, wobei die Suspension der Kapseln einen Anteil von Kapseln aufweist, der im Bereich von 3 bis 90 Gew.-%, insbesondere im Bereich von 10 bis 60 Gew.-% und noch spezieller im Bereich von 20 bis 50 Gew.-% in Bezug auf das Gesamtgewicht der Suspension liegt.

5. Verfahren nach einem der vorhergehenden Ansprüche, wobei die Polymere und die ölige Phase in einem Gewichtsverhältnis vorliegen, das im Bereich von 5/1 bis 1/20, insbesondere im Bereich von 2/1 bis 1/20 und noch spezieller im Bereich von 2/1 bis 1/10 liegt.

6. Verfahren nach einem der vorhergehenden Ansprüche, wobei die ölige Phase wenigstens ein fettlösliches aktives Mittel und/oder ein fettdispersibles aktives Mittel enthält.

7. Verfahren nach einem der vorhergehenden Ansprüche, das im Schritt a) Polymere mit einem Molekulargewicht größer oder gleich 10000, insbesondere größer oder gleich 15000 enthält.

8. Verfahren nach einem der vorhergehenden Ansprüche, wobei die Polymere einen Schmelzpunkt P_{f} kleiner als 100 °C besitzen, der insbesondere im Bereich von -20 °C bis 95 °C und noch spezieller im Bereich von -10 °C bis 80 °C liegt.

9. Verfahren nach einem der vorhergehenden Ansprüche, wobei das oder die Oligomere gewählt sind aus Polycaprolactonen, Poly-L-Lactiden, Poly-DL-Lactiden, Polyglycoliden und ihren Copolymeren, Polymeren von 3-Hydroxy-Buttersäure und ihren Polymeren mit hydroxyvalerischer Säure, Diol-Polycarbonaten, Poly(Alklyen-Adiabat), Polyol-Polyestern, dendritischen Polyestern mit Hydroxylabschlussfunktion und ihren Gemischen.

10. Verfahren nach einem der vorhergehenden Ansprüche, wobei die ölige Phase ein aktives Mittel bildet.

11. Verfahren nach einem der vorhergehenden Ansprüche, wobei die ölige Phase wenigstens ein Öl enthält, das aus nichtflüchtigen Ölen und Gemischen hiervon gewählt ist.

12. Verfahren nach einem der vorhergehenden Ansprüche, wobei wenigstens ein grenzflächenaktives Mittel in öliger Phase und/oder wässriger Phase eingesetzt wird.

13. Verfahren nach einem der Ansprüche 2 bis 12, wobei der Schritt g) der Vernetzung ein Vernetzungsmittel verwendet, das aus den difunktionalen oder polyfunktionalen hydrophilen oder lipophilen Molekülen gewählt ist.

14. Verfahren nach dem vorhergehenden Anspruch, wobei die Funktionen des Vernetzungsmittels, die gleich oder verschieden sind, aus Isocyanat, gemischtem Anhydrid und Säurechlorid gewählt sind.

15. Verwendung einer Suspension von Kapseln, die gemäß dem Verfahren nach einem der Ansprüche 1 bis 14 erhalten wird, um eine kosmetische, dermatologische oder pharmazeutische Zusammensetzung zuzubreiten.

16. Kosmetische, dermatologische oder pharmazeutische Zusammensetzung, die in einem physiologisch annehmbaren Milieu eine Suspension von Kapseln enthält, die gemäß dem Verfahren nach einem der Ansprüche 1 bis 14 erhalten werden kann.

17. Kapseln, die mittels des Verfahrens nach einem der Ansprüche 1 bis 14 erhalten werden können.

18. Wässrige Lösung von Kapseln, die mittels des Verfahrens nach einem der Ansprüche 1 bis 14 erhalten werden können.
